(19) Europäisches Patentamt
European Patent Office
Office européen des brevets

(11) **EP 2 967 712 B1**

(12) **EUROPÄISCHE PATENTSCHRIFT**

(45) Veröffentlichungstag und Bekanntmachung des Hinweises auf die Patenterteilung:
**08.03.2017 Patentblatt 2017/10**

(51) Int Cl.:
*A61B 18/12* (2006.01)     *A61B 18/14* (2006.01)
*A61F 2/95* (2013.01)

(21) Anmeldenummer: **14709301.7**

(22) Anmeldetag: **11.03.2014**

(86) Internationale Anmeldenummer:
**PCT/EP2014/054740**

(87) Internationale Veröffentlichungsnummer:
**WO 2014/140039 (18.09.2014 Gazette 2014/38)**

(54) **CHIRURGISCHER GLEICHSTROMSCHNEIDER**

SURGICAL DIRECT CURRENT CUTTER

DISPOSITIF DE DÉCOUPE CHIRURGICALE AU COURANT CONTINU

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.03.2013 DE 102013102418**

(43) Veröffentlichungstag der Anmeldung:
**20.01.2016 Patentblatt 2016/03**

(73) Patentinhaber: **Ovesco Endoscopy AG**
**72074 Tübingen (DE)**

(72) Erfinder:
• **SCHOSTEK, Sebastian**
**72074 Tübingen (DE)**
• **HO, Chi-Nghia**
**72762 Reutlingen (DE)**
• **MELBERT, Michael**
**72127 Kusterdingen-Immenhausen (DE)**
• **SCHURR, Marc O.**
**72072 Tübingen (DE)**
• **GOTTWALD, Thomas**
**82431 Kochel (DE)**

(74) Vertreter: **Winter, Brandl, Fürniss, Hübner, Röss, Kaiser, Polte - Partnerschaft mbB Patent- und Rechtsanwaltskanzlei Alois-Steinecker-Strasse 22 85354 Freising (DE)**

(56) Entgegenhaltungen:
**EP-A1- 2 392 282       WO-A2-2008/040485**
**DE-A1-102007 003 838   US-A- 3 354 478**
**US-A- 3 431 384        US-A1- 2012 265 196**
**US-A1- 2013 030 428**

• **Anonymous: "Wolfram - Wikipedia", , 5. März 2013 (2013-03-05), XP055122926, Gefunden im Internet: URL:http://de.wikipedia.org/w/index.php?title=Wolfram&oldid=114988807 [gefunden am 2014-06-12]**

**Beschreibung**

Die vorliegende Erfindung betrifft eine chirurgische Schneideinrichtung für das Fragmentieren dünnwandiger und/oder drahtförmiger, metallischer Implantate mit Gleichstrom.

In der Endoskopie werden zur Therapie beispielsweise von Verengungen, Perforationen und Fisteln insbesondere im Verdauungstrakt eines Patienten häufig sogenannte Stents eingesetzt. Derartige Stents sind rohrförmige Geflechte, die, auf Minimalgröße zusammengestaucht oder gefaltet, über ein eingeführtes Endoskop oder einen Trokar zu einem ausgewählten Zielort geführt und dann appliziert werden. Nach der Applikation entfaltet sich der jeweilige Stent elastisch zu seiner vollständigen Größe und kann so die Organwand stützen bzw. vorhandene Fisteln oder Perforationen abdecken und verschließen.

Jene Stents, welche zur Abdeckung von Organwandperforationen oder Fisteln vorgesehen sind, haben zwischen ihrem Drahtgeflecht häufig eine Silikonmembran. Damit wird verhindert, dass u. a. Verdauungssäfte und/oder Bakterien in die Perforation bzw. Fistel gelangen und dort zu Entzündungen führen oder zumindest den Heilungsprozess behindern / verhindern. Nach Ausheilung der Läsion kann in der Regel ein solcher Stent wieder aus dem Körper geborgen werden, wofür es im Stand der Technik eine Mehrzahl an Bergevorrichtungen gibt.

Stents dieser Art können jedoch insbesondere dann, wenn sie über einen längeren Zeitraum im Körper verbleiben müssen, in das Organgewebe einwachsen, sodass sie zum Teil mit Gewalt aus dem Gewebe herausgerissen werden müssen, wodurch naturgemäß Komplikationen auftreten können, wie z.B. die erneute Erzeugung einer Läsion und im schlimmsten Fall einer Perforation.

Dabei betreffen nachträglich zu entfernende Implantate nicht nur geflechtartige Stents gemäß vorstehender Beschreibung sondern auch Gewebeclips zur temporären Verankerung von diversen Messsonden in einem Hohlorgan oder zum punktuellen Verschließen von Organperforationen, wie sie beispielsweise beim Entfernen von Polypen Verdauungstrakt eines Patienten entstehen können. Auch existieren hakenförmige Gewebeanker/Spreizanker, deren Tentakel sich in ein Organgewebe bohren und dann bereits nach kurzer Zeit einwachsen können.

Um Implantate dieser Art für den Patienten gefahrlos explantieren zu können, besteht die grundsätzliche Notwendigkeit für ein Instrument, welches das betreffende Implantat durch Schneiden (Zerschneiden) des Implantatmaterials leichter entfernbar macht und so möglichen Komplikationen vorbeugt. Da jedoch, wie vorstehend bereits angedeutet wurde, derartige Implantate zumeist aus Metall oder einer metallischen Legierung bestehen, die in der Regel ausgelegt sind, besondere Belastungen auszuhalten, sind mechanische Schneidwerkzeuge wie sogenannte Endoskopscheren, HF-Schlingen, APC, etc. eigentlich unbrauchbar, zumindest jedoch ungeeignet.

Es hat sich jedoch gezeigt, dass nur ein solches chirurgisches Instrument für derartige besondere Zwecke (Explantieren metallischer Implantate) eingesetzt werden kann, welches es ermöglicht, das Implantatsmaterial vorzugsweise segmentweise durch ein partielles Aufschmelzen zu zerstören (zu fragmentieren). Hierfür wurden im Stand der Technik bereits HF-Instrumente der Bipolarbauart vorgeschlagen, von denen eines beispielsweise aus der WO 2008/090003 A1 bekannt ist.

Das hierin offenbarte bipolare Instrument dient zur endoskopisch kontrollierten Kürzung und/oder Fragmentierung von im Gastrointestinaltrakt, im Tracheobronchialsystem oder in anderen Hohlorganen befindlichen Stents und hat an seinem distalen Instrumentenkopf zwei fest miteinander verbundene, d.h. relativ zueinander unbewegliche Instrumentenbranchen, welche zwischen sich einen in Richtung proximal V-förmig verjüngenden Spalt definieren. In einem Axialabstand zum proximalen Ende des V-förmigen Spalts befindet sich eine Elektrode, welche zum Instrumentenkopf bzw. zu den Instrumentenbranchen in elektrisch isolierender Weise gelagert ist.

Wird nunmehr der Instrumentenkopf beispielsweise gegen ein Stentgeflecht bewegt, gelangt der Flechtdraht in den V-förmigen Spalt zwischen die Instrumentenbranchen, bis dieser am spalthintersten Ende zu liegen kommt. In dieser Position hat der Flechtdraht dann einen optimalen Abstand zur Elektrode, wodurch sich bei einer HF-Strombeaufschlagung ein Lichtbogen zwischen der Elektrode und dem Flechtdraht ausbildet, der den Flechtdraht zum Schmelzen bringt.

Alternativ zu der Ausbildung eines Lichtbogens wird in diesem Stand der Technik auch angeregt, die Elektroden unmittelbar mit dem Stentmaterial / Draht in Körperkontakt zu bringen und somit direkt Strom in den Stentdraht für dessen Erwärmung einzuleiten. An Stelle des bevorzugten HF-Wechselstroms kann es gemäß diesem Stand der Technik aber auch vorgesehen sein, die Elektrode zur Ausbildung eines Lichtbogens mit einem Gleichstrom oder einem niederfrequenten Wechselstrom zu beaufschlagen.

Das grundsätzliche Problem dieses bekannten Instruments stellt zunächst die Ausbildung eines geeigneten Lichtbogens zwischen der Schweißelektrode und dem Stentmaterial dar. Bei unterschiedlichen Materialstärken des Implantats kann sich nämlich der Abstand zur Elektrode verändern bzw. unterschiedlich einstellen, wodurch die Lichtbogenausbildung beeinflusst wird. Damit kann das Schneidevermögen des bekannten Instruments mit starren Instrumentenbranchen nicht für alle bekannten Implantate stabil aufrecht erhalten werden.

Die DE 10 2007 003838A1 offenbart ein bipolares Instrument und ein Verfahren zur endoskopisch kontrollierten Kürzung und/oder Fragmentierung von im Gastrointestinaltrakt, im Tracheobronchialsystem oder in anderen Hohlorganen befindlichen Stents. Das Instrument umfasst eine an einem distalen Ende des Instruments angeordnete Elektrodeneinrichtung mit mindestens einer ersten Elektrode und einer zweiten Elektrode zum Durchleiten eines Stromes von einer Stromquelle durch mindestens einen Draht des Stents und/oder zum Bilden elektrischer Lichtbogen zwischen der ersten Elektrode und dem mindestens einen Draht und/oder zwischen der ersten Elektrode und der zweiten Elektrode, so dass der Draht durch Erhitzen und Schmelzen durchtrennbar ist.

Die US 3431384 A offenbart eine Vorrichtung, die Drähte mittels Strompulsen schmelzen und somit trennen kann.

Die WO 2008/040485 A2 offenbart ein medizinisches Instrument, insbesondere ein Rohrschaftinstrument, zum Schneiden von Gewebe. Weiter wird offenbart, dass Elektroden an dem Instrument vorgesehen sind, wodurch ein mechanisches Kontaktieren zwischen einer Schneide und einer zugehörigen Schneidfläche auf elektrischem Weg feststellbar ist.

Die US 2012/265196 A1 zeigt eine Steuerschaltung einer chirurgischen Vorrichtung. Die Steuerschaltung umfasst einen ersten Schaltungsabschnitt mit zumindest einem Schalter.

Die US 2013/030428 A1 offenbart ein chirurgisches Rohrschaftinstrument mit Elektroden an den proximalen, zangenartigen Instrumentenbranchen und einer speziellen Steuerung des Elektrodenstroms (Multiphasen).

Die EP 2 392 282 A1 offenbart ein Gerät zur Durchführung einer chirurgischen Prozedur. Insbesondere offenbart dieses Dokument ein elektrochirurgisches Instument mit einem End effector der ein Paar Backenteile aufweist.

Ein weiteres grundlegendes Problem ist der Wärmeeintrag in das Stentmaterial. Wird nämlich elektrischer Strom beispielsweise direkt in das Stentmaterial eingeleitet, erhitzt sich dieses, was zu Schädigungen des umliegenden Patientengewebes führen kann. Aus diesem Grund sieht dieser Stand der Technik auch eine Schutzvorrichtung vor, welche die Elektroden / den Stentdraht vom Patientengewebe beabstandet. Eine solche Schutzvorrichtung verteuert das Instrument und macht es auch unhandlicher.

Angesichts dieser Problematik ist es die Aufgabe der vorliegenden Erfindung, einen auf Gleichstrombasis arbeitenden chirurgischen Implantatschneider bereit zu stellen, der endoskopisch oder nach Art eines Katheters in den Patienten einführbar ist, und dessen Schneidvermögen für unterschiedliche Implantate stabil gehalten werden kann. Ein bevorzugtes Ziel der vorliegenden Erfindung ist es zudem, den chirurgischen Implantatschneider möglichst kostengünstig ohne größeren konstruktiven Aufwand zu gestalten. Schließlich sollte der erfindungsgemäße chirurgische Implantatschneider vorzugsweise Anwendungsfehler vermeiden helfen oder zumindest anzeigen.

Diese Aufgabe sowie die bevorzugten weiteren Ziele werden durch einen Implantatschneider mit den Merkmalen des Anspruchs 1 gelöst. Vorteilhafte Ausgestaltungen sind Gegenstand der Unteransprüche.

Die Erfindung basiert dabei auf den folgenden grundsätzlichen Überlegungen:

- Um eine Gewebeschädigung auch bei Fehlen einer speziellen Schutzvorrichtung zu vermeiden, sollte der Wärmeeintrag möglichst gering sein und dennoch zu einem Aufschmelzen des Stentmaterials führen. Dies kann erreicht werden, indem eine Kontakt-/Berührungsfläche zwischen Elektrode und Stentmaterial möglichst klein gehalten ist (vorzugsweise punktförmig) um im Kontaktbereich / Übergang eine hohe Stromdichte (bei direktem Anlegen eines Gleichstroms) zu erhalten. D.h. die aktive Kontakt-/Berührungsfläche an zumindest einer Elektrode ist so gestaltet, dass hier die höchste Stromdichte längs des gesamten elektrischen Strompfads erzielt wird. Diese reicht dann aus, das Stentmaterial (nur) an der Kontaktstelle partiell aufzuschmelzen, ohne dass sich der gesamte Stent über Gebühr aufheizt.

- Zusätzlich hierzu sollte der Wärmeeintrag durch direktes Anlegen eines elektrischen Gleichstroms dadurch erfolgen, indem der elektrische Gleichstrom gepulst oder getaktet angelegt wird. Jedes daraus sich ergebende Gleichstrompaket bewirkt zwar eine Wärmeenergiezufuhr in das Stentmaterial, wobei jedoch der Wärmeabfluss in das umliegende Gewebe aufgrund der zwangsläufig kurzen Impulsdauer vergleichsweise (gegenüber nichtgetakteter Strombeaufschlagung) nur gering ist.

- Die Elektroden des bipolaren Instruments sollten zusätzlich oder alternativ hierzu an der Instrumentenspitze so angeordnet sein, dass das Instrument selbst eine Art Schutzwirkung entfaltet, ohne dass eine spezielle Schutzvorrichtung gemäß dem Stand der Technik angeordnet werden müsste. Hierfür hat sich die Ausbildung zweier in Instrumentenlängsrichtung sich erstreckender, zwischen sich einen (Längs-) Schneidspalt ausbildender Instrumentenbranchen als vorteilhaft erwiesen, die an ihren jeweils sich zugewandten Seiten mit Elektroden besetzt sind oder Elektroden ausbilden. Damit wenden sich die Elektroden zwangsläufig vom umliegenden Gewebe ab, wobei sich die Branchen zwischen den Elektroden und dem umliegenden Gewebe abschirmend anordnen.

- Um den wirksamen Energieeintrag in das Stentmaterial auf ein Minimum zu beschränken, ist es vorteilhaft, diesen zu kennen bzw. zu bestimmen. Daher kann zusätzlich oder alternativ zu den vorstehend genannten Maßnahmen eine Messvorrichtung vorgesehen sein, welche vorzugsweise den elektrischen Widerstand am Übergangs-/Kontaktbereich zwischen Elektrode und Stentmaterial bestimmt und daraus jenen elektrischen Stromwert festlegt, der für ein partielles Aufschmelzen des Stentmaterials ausreicht.

- Zusätzlich oder alternativ hierzu hat das Elektrodenmaterial auch eine bestimmte Materialeigenschaft,
welche einem thermischen Materialverschleiß entgegenwirkt bzw. diesen verhindert/einschränkt.
Hierzu wird an dieser Stelle der Begriff der "Widerstands - adjustierten spezifischen Schmelzenergie"
als die bestimmte Materialeigenschaft in diese Anmeldung eingeführt. Diese berechnet sich im Wesentlichen aus materialspezifischen Konstanten wie
der spezifischen Wärmekapazität c, der Massendichte p, der Schmelztemperatur $T_S$ und dem spezifischen elektrischen Widerstand r. Diese Materialeigenschaft lässt einen direkten Vergleich verschiedener Materialien dahingehend zu, wie schnell
diese Materialien bei einem gegebenen elektrischen
Stromfluss aufgeschmolzen werden (je niedriger die
Widerstands-adjustierte spezifische Schmelzenergie, desto schneller wird ein Material bei gegebenem
Stromfluss aufgeschmolzen). Der Wert der Widerstands - adjustierten spezifischen Schmelzenergie
für das Elektrodenmaterial soll erfindungsgemäß höher sein als das des Stentmaterials, vorzugweise um
mindestens den Faktor 2.

Ein Aspekt der vorliegenden Erfindung besteht
nunmehr darin, dass für den Fall, dass die sich gegenüberliegenden, einen Schneidspalt ausbildenden Instrumentenbranchen jeweils mit einer Elektrode bestückt
sind oder jeweils eine Elektrode ausbilden, das zu fragmentierende Implantatsmaterial bei dessen Einführen in
den Schneidspalt unmittelbaren elektrischen sowie körperlichen Kontakt mit den Elektroden erhält (also diese
kurzschließt), wodurch ein Gleichstrom (Kurzschlussstrom) durch das Implantatsmaterial (ohne Lichtbogenbildung) geleitet wird, das zu dessen partieller Erhitzung
führt. D.h. das zwischen den Elektroden befindliche Implantatsmaterial wird bei entsprechend gewählter Stromstärke stark erhitzt und beginnt partiell (zwischen den
Elektroden) aufzuschmelzen und zu zerfließen/abzutropfen. Wird dabei der Instrumentenkopf ggf. weiter in
das Implantatmaterial hineinbewegt, bewirken die beiden Instrumentenbranchen ggf. zusätzlich ein Zerteilen
des aufgeschmolzenen Implantatmaterials bei geringer
mechanischer Vorschubkraft, welche über den Instrumentenschaft beispielsweise innerhalb der Einführhilfe
(Endoskop, Trokar, etc.) problemlos aufgebracht werden
kann.

Bei dieser Anordnung ist die Ausbildung eines
Lichtbogens sowie der konstruktive Aufwand zur Einstellung eines korrekten Abstands zwischen dem Implantatmaterial und einer Elektrode gemäß dem bekannten
Stand der Technik nicht erforderlich, wodurch sich das
Instrument wesentlich einfacher und damit preisgünstiger fertigen lässt. Darüber hinaus unterscheidet sich der
vorliegende Erfindungsgegenstand auch grundlegend
von an sich bekannten TFT - Instrumenten der Bipolarbauart (Tissue Fusion Instruments), bei denen zwar
ebenfalls zwei Instrumentenbranchen mit Elektroden
zum Gewebezerteilen oder Gewebeschweißen (Koagulation) bestückt sind, die Elektroden jedoch zum Einen
mit HF-Strom beaufschlagt werden und zum Anderen die
Instrumentenbranchen relativ zueinander bewegbar sein
müssen, um das zu behandelnde Patientengewebe zwischen sich mit einem bestimmten Anpressdruck einzuspannen. Im Gegensatz hierzu wird aber das metallische
Implantatmaterial erfindungsgemäß durch den Gleichstromkurzschluss zumindest soweit aufgeschmolzen,
dass es beim Vorwärtsschieben des Instrumentenkopfs
durch die Instrumentenbranchen einfach zerteilt werden
kann.

Des Weiteren ist es eine durch die Dimensionierung und Gestaltung der Elektroden zu erfüllende Voraussetzung, dass sich im maßgeblichen Kontaktschluss
zwischen den Elektroden bzw. zumindest einer der Elektroden und dem Implantat eine besonders hohe Stromdichte infolge eines dort sich ergebenden örtlich reduzierten Strompfads ausbildet. D.h. die Aufstandsfläche
und damit der Strompfad zwischen der zumindest einen
Elektrode und dem Implantat muss so klein sein, dass
die dort entstehende hohe Stromdichte zu einem Aufschmelzen des Implantatmaterials (nur) zwischen den
Elektroden führt. Erreicht wird dies, indem die zumindest
eine Elektrode (oder die Elektroden an beiden Branchen)
einen für den Kontakteingriff mit dem Implantat vorgesehenen Bereich hat, der bezogen auf eine Ebene (d.h. bei
Aufliegen der Elektrode auf einer planen Fläche) eine
(im wesentlichen eindimensionale) Kontaktlinie vorzugsweise in Elektrodenlängsrichtung oder einen (im wesentlichen eindimensionalen) Kontaktpunkt aufzeigt bzw. definiert. Eine solche Kontaktlinie ergibt sich beispielsweise
dann, wenn die jeweilige Elektrode mit einer Schneidkante versehen ist oder eine solche Schneidkante bildet.
Es ist auch denkbar, dass die zumindest eine Elektrode
an ihrer der anderen Elektrode zugewandten Seite konvex oder rinnenförmig nach außen längsgewölbt ist. Wird
eine solche Rinne an ihrem Außenumfang auf eine Ebene aufgelegt, ergibt sich hieraus zweifelsfrei ein (im wesentlichen eindimensionaler) Punktkontakt. Auch besteht die Möglichkeit, die zumindest eine Elektrode mit
einer schmalen Längswulst auszubilden, die eine zur anderen Elektrode vorstehende (scharfkantige) Kontaktleiste an der Elektrode bildet. Schließlich ist es auch
denkbar, die zumindest eine Elektrode kugelkalottenförmig zu gestalten, um so einen im wesentlichen Punktkontakt zu verwirklichen/anzunähern.

Um das umliegende Patientengewebe nicht zu
beschädigen, wäre es vorteilhaft, wenn die sich gegenüberliegenden Elektroden, zwischen denen sich der
Schweißspalt in Instrumentenlängsrichtung ausbildet, in
Bruchteilen einer Sekunde mit Gleichstromimpulsen hoher Stromstärke (von bis zu 200 Ampere, vorzugsweise
zwischen 100 - 150 A) steuerungstechnisch beaufschlagt werden. Das Metall wird dadurch zwischen den
Elektroden aufgeschmolzen und zerschnitten, ohne dass sich das Implantatmaterial in der Umgebung zum Schneidspalt übergebührlich erwärmt. Der Grund hierfür besteht, wie vorstehend bereits angedeutet wurde, darin, dass durch Strompulsung der Wärmeabflusseffekt vom Stent in das umliegende Gewebe verringert werden kann. Die Spannung kann dabei weit unter einem Grenzwert von 48 Volt für eine Niederspannung liegen (vorzugsweise zwischen 20 - 40 V), die für den Patienten vollkommen unbedenklich ist.

Natürlich besteht die Möglichkeit, die beiden Instrumentenbranchen und/oder die daran montierten Elektroden federelastisch nachgebend zu gestalten / zu lagern, sodass auch Implantate mit größeren Materialstärken in den Schweißspalt einführbar sind und sicher an den Elektroden anliegen. Dabei kann es sogar vorgesehen sein, die Branchen über einen Betätigungsmechanismus zu bewegen, um den Schweißspalt (Spaltweite) einzustellen.

Um das Implantatmaterial mit ausreichender Sicherheit - auch für den Patienten - aufschmelzen und zerteilen zu können, wäre es vorteilhaft, wenn die Branchen und/oder Elektroden an ihren jeweils zugewandten Längsseiten zu einer schmalen Schnittkante oder Noppe als alleinig wirksame Kontaktfläche geformt sind, um nahezu Punktkontakt mit dem Implantatmaterial zu erzielen. An diesen sehr kleinflächigen Kontaktstellen erhitzt sich das Implantatsmaterial aufgrund der hohen Stromdichte besonders auf und lässt sich somit schnell aufschmelzen, bevor das weiter weg gelegene Implantatmaterial aufgewärmt wird. Hierfür hat es sich als besonders vorteilhaft erwiesen, wenn zumindest die Elektroden aus einem hitzebeständigen Material wie Wolfram gefertigt sind oder aus einem niedrig legierten Stahl bestehen.

Um den Zerteilvorgang zu optimieren, können die beiden Elektroden und/oder die beiden Instrumentenbranchen im Wesentlichen V-förmig zueinander ausgerichtet sein, sodass sich der dazwischen sich ausbildende Schweiß-/Schneidspalt in Richtung proximal kontinuierlich linear oder in konvexer Kurvenform verengt.

Auch kann eine Art Vorspanneinrichtung an der Instrumentenspitze vorgesehen sein, welche die Elektroden und/oder die Instrumentenbranchen mit einer bestimmten Vorspannkraft gegeneinander vorspannt, sodass ein dazwischen eingeführtes Implantatmaterial beidseits quasi automatisch zusammengedrückt / gequetscht wird. Aus der vorstehenden Beschreibung lässt sich entnehmen, dass für ein sicheres Zerteilen eines metallischen Implantatmaterials die Greif- bzw. Kontaktqualität zwischen Implantatmaterial und Elektrode eine große Rolle spielt. Je kleiner nämlich die Kontaktfläche zwischen Implantat und Elektroden ist, desto schneller schmilzt das Implantatsmaterial im Kontaktbereich zwischen den Elektroden auf und desto geringer kann der Energieeintrag in das Stentmaterial sein. Daher ist erfindungsgemäß eine elektrische / elektronische Kontaktqualitätserkennung (Messvorrichtung) optional vorgesehen, die bei einer Betätigung des Instruments sowie (zeitlich) vor der daraus resultierenden Elektrodenbeaufschlagung mit dem Leistungsgleichstrom / Schneidstrom (automatisch immer oder selektiv) eine Testsequenz ausführt. In diesem Fall wird unter Beaufschlagung der Elektroden beispielsweise mit einem sehr kleinen Teststrom deutlich unterhalb des Schweiß- / Schneidstroms oder mit dem Leistungsstrom dann jedoch für eine extrem kurze Impulszeit, sodass sich das Implantatgewebe nicht oder vernachlässigbar erwärmt, der Kontaktwiderstand zwischen den jeweiligen Elektroden und dem Implantatmaterial ermittelt und für den Fall des Unterschreitens eines Widerstands - Grenzwerts (zu kleiner Kontakt - Widerstand) der Benutzer gewarnt und/oder die nachfolgende Leistungsgleichstrom - Beaufschlagung (Schneidstrombeaufschlagung) blockiert. Zusätzlich oder alternativ kann die Höhe des zuzuführenden Leitungsgleichstroms anhand des Bestimmungsergebnisses so angepasst werden, dass ein Aufschmelzen des Implantatmaterials gewährleistet ist. Ferner kann in der Testsequenz auch nachgeprüft werden, ob der erforderliche Leistungsgleichstrom die Belastbarkeit der Zuführleitungen und/oder der Schneidelektroden übersteigen würde, um so einer Beschädigung oder einem schnellen Verschleiß des Schneidinstruments vorzubeugen.

In der Regel sind Stentimplante aus einem Edelstahl oder einer Titanlegierung (Martensit) gefertigt. Erfindungsgemäße Untersuchungen ergaben für die allgemein bekannten gängigen Stentmaterialien eine Widerstands - adjustierte spezifische Schmelzenergie von zwischen 4.86 bis 5.99 $10^{15}$ J m$^{-4}$ $\Omega^{-1}$. Demzufolge sieht ein weiterer, ggf. unabhängig zu beanspruchender Aspekt der Erfindung eine Widerstands - adjustierte spezifische Schmelzenergie für das Elektrodenmaterial größer als 5.99 $10^{15}$ J m$^{-4}$ $\Omega^{-1}$ vorzugsweise um min. den Faktor 2 vor. Dadurch wird einem schnellen thermischen Verschleiß des Elektrodenmaterials entgegengewirkt.

Die Erfindung wird nachstehend anhand bevorzugter Ausführungsbeispiele unter Bezugnahme auf die begleitenden Figuren näher erläutert.

Der Schutzanspruch wird durch den beigefügten Hauptanspruch 1 definiert. Bevorzugte Ausführungsbeispiele sind in den abhängigen Ansprüchen gegeben.

Die zweiteilige Form des Anspruchs 1 basiert auf DE 10 2007 003838.

Fig. 1 zeigt die konzeptionelle Anordnung eines mit Gleichstrom betriebenen chirurgisches Implantatschneideinstruments der Bipolarbauart gemäß einem bevorzugten Ausführungsbeispiel der vorliegenden Erfindung,

Fig. 2 zeigt die konzeptionelle Ausbildung zweier rigider Instrumentenbranchen an einem distalen Instrumentenkopf,

Fig. 3a und 3b zeigen die konzeptionelle Ausbildung zweier elastischer oder elastisch gelagerter Instrumentenbranchen am distalen Instrumentenkopf in einer aus einer Einführhilfe ausgefahrenen und zurückgezogenen Betriebsposition,

Fig. 4a und 4b zeigen die konzeptionelle Ausbildung zweier gelenkig gelagerter Instrumentenbranchen am distalen Instrumentenkopf mit aufgeweitetem und eingeengtem Schneidspalt,

Fig. 5 zeigt die konzeptionelle Ausbildung zweier Instrumentenbranchen am distalen Instrumentenkopf im Kontakteingriff (Kurzschlusseingriff) mit einem elektrisch leitenden Implantat,

Fig. 6 zeigt das Greif- oder Kontakteingriffsprinzip zwischen dem erfindungsgemäßen Schneidinstrument und dem elektrisch leitenden Implantat,

Fig. 7 zeigt ein Vergleichsdiagramm zwischen der Widerstands-adjustierten spezifischen Schmelzenergie und der jeweiligen Stromdichte längs eines Instrumenten - Implantat - Strompfads,

Fig. 8 zeigt ein Widerstandsschaltbild des Instrumenten - Implantat - Strompfads für eine Testsequenz einer elektrischen / elektronischen Kontaktqualitätskennung der Instrumentensteuerung,

Fig. 9 zeigt ein Widerstandsschaltbild des Instrumenten - Implantat - Strompfads für eine Leistungsstrombeaufschlagung (Schneidsequenz) der Instrumentensteuerung,

Fig. 10a bis 10e zeigen ein bevorzugtes Ausführungsbeispiel für die Konstruktion der Instrumentenspitze und/oder der Instrumentenbranchen / Elektroden,

Fig. 11 zeigt das Grundprinzip eines elektrischen Strompfads durch ein metallisches Implantat, an das zwei Elektroden in Gegenüberlage punktförmig angelegt sind,

Fig. 12 zeigt den elektrischen Strompfad gemäß Fig. 11 unter Zuordnung der zu erwartenden Stromdichten in einzelnen Pfadabschnitten,

Fig. 13 zeigt den elektrischen Strompfad gemäß Fig. 11 sowie die thermische Auswirkung auf ein Implantat im Übergangs-/Kontaktbereich zwischen Implantat und Elektroden und

Fig. 14 zeigt eine Tabelle bezüglich der Energieaufnahmeeigenschaften unterschiedlicher elektrisch leitender Materialien.

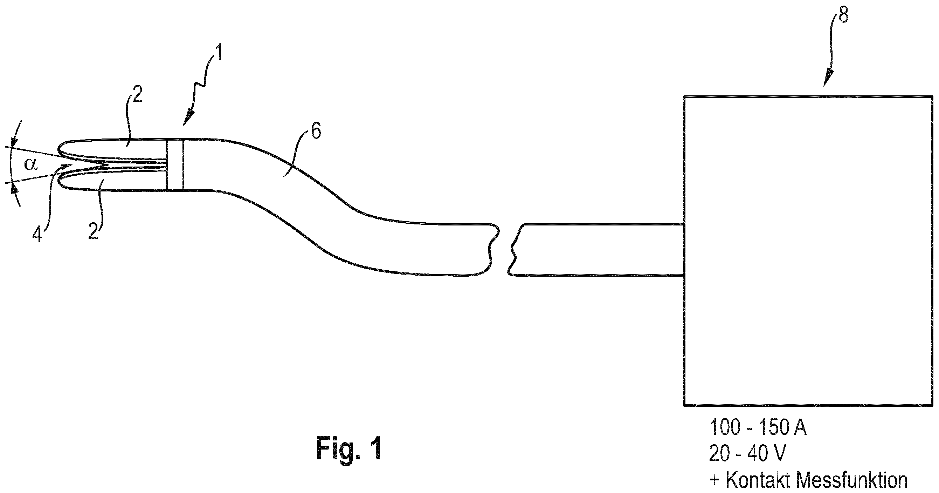

Das in der Fig. 1 schematisch dargestellte chirurgische Implantat - Schneidinstrument der Bipolarbauart hat einen distalen Instrumentenkopf oder eine Instrumentenspitze 1 mit (zumindest) zwei Instrumentenbranchen 2, welche zwischen sich einen in Instrumentenlängsrichtung sich erstreckenden Längsspalt 4 definieren. Der Instrumentenkopf 1 ist an einem distalen Ende eines vorzugsweise biegeflexiblen (oder auch starren), zumindest 2-poligen Instrumentenschafts 6 montiert, der wiederum an einen gesteuerten / geregelten bzw. steuer-/regelbaren Gleichstromgenerator 8 angeschlossen ist. Der Gleichstromgenerator 8 liefert einen Gleichstrom von vorzugsweise wahlweise zwischen 100A- 150A bei einer Spannung von vorzugsweise wahlweise 20V - 40V.

Wie ferner aus der Fig. 1 zu entnehmen ist, hat der zwischen den Instrumentenbranchen 2 sich ausbildende Spalt im Wesentlichen eine in Richtung proximal sich verjüngende V-Form. Der Instrumentenschaft 6 und auch der Instrumentenkopf 1 haben einen solchen Außendurchmesser (beispielsweise max. 6mm), der es ermöglicht, dass das chirurgische Instrument in den (standardisierten) Arbeitskanal eines per se bekannten Endoskops, Trokars oder dergleichen Einführhilfe einführbar ist. Alternativ kann der Instrumentenschaft und Instrumentenkopf so gestaltet und dimensioniert sein, dass er nach Art eines Katheters (ohne Einführhilfe) in ein Patentenhohlorgan eingeführt werden kann.

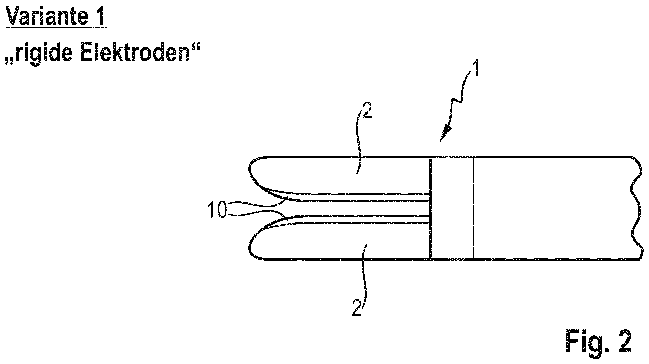

Die Fig. 2 zeigt in Vergrößerung eine Variante für eine erfindungsgemäße Branchenkonstruktion.

Demnach sind die Instrumentenbranchen 2 fest mit dem Instrumentenkopf 1 verbunden und zudem starr ausgeführt. An den einander zugewandten Längsseiten jeder Branche 2 sind elektrisch leitende Elektroden 10 angeordnet, die über zwei Stromleiter (nicht dargestellt) innerhalb des Instrumentenschafts 6 mit dem Gleichstromgenerator 8 verbunden sind. Die Branchen 2 können dabei selbst die Elektroden 10 bilden, wofür sie jedoch elektrisch isoliert am Instrumentenkopf 1 fixiert sein müssen. Alternativ können die Instrumentenbranchen 2 jedoch auch mit externen Elektroden 10 ausgerüstet/bestückt sein. Die Ausbildung/Anordnung der Elektroden 10 sowie deren Verbindung an den Gleichstromgenerator ist bei allen nachfolgend noch beschriebenen Varianten gleich, sodass diese nicht in sich wiederholender Weise beschrieben wird.

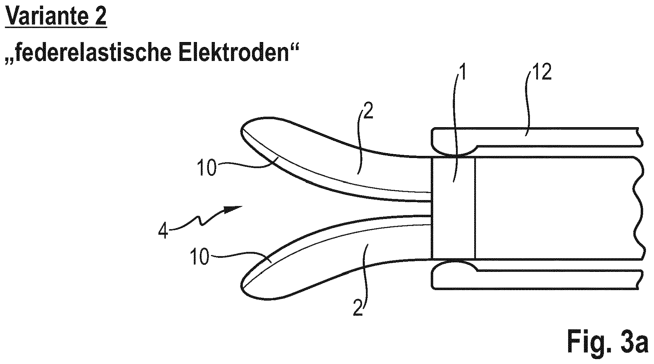

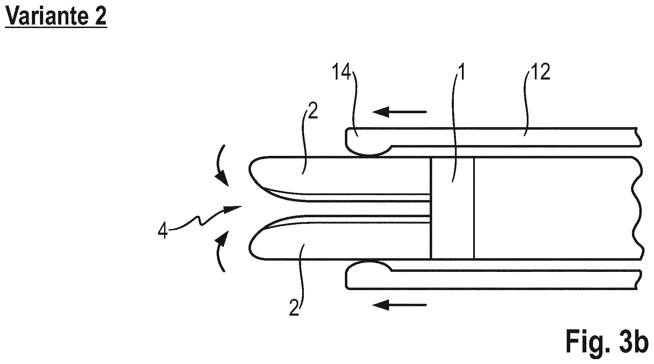

Gemäß der in Fig. 3 gezeigten Variante sind die Branchen 2 und/oder die Elektroden 10 aus einem biegeflexiblen, vorzugsweise elastischen Material gefertigt oder die Elektroden 10 sind federnd an den jeweiligen Instrumentenbranchen 2 gelagert, derart, dass sie unter Vergrößerung der Spaltweite in die Branchen 2 elastisch eingedrückt werden können.

Gemäß der Fig. 3 ist zumindest am distalen Endbereich des Instruments eine Außenhülse 12 vorgesehen, die axial relativ zum Instrument bewegbar ist. Diese Hülse 12 kann ein separates Bauteil oder die Einführhilfe selbst sein. Dabei ist das Instrument in einer Position dargestellt, in der der Instrumentenkopf 1, zumindest jedoch die Instrumentenbranchen 2 axial aus der Hülse 12 vorragen und sich dabei radial (elastisch) aufspreizen, wodurch sich der Schneidspalt 4 aufweitet.

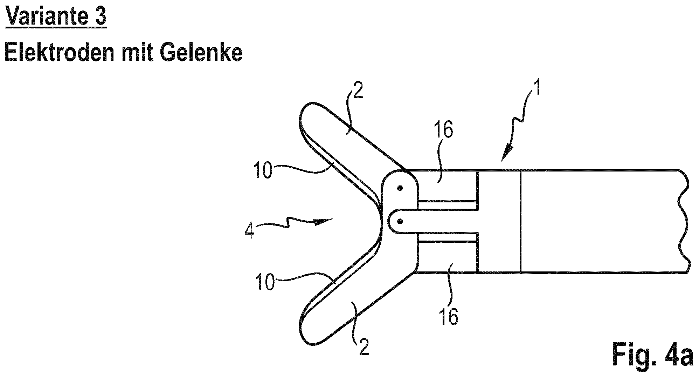

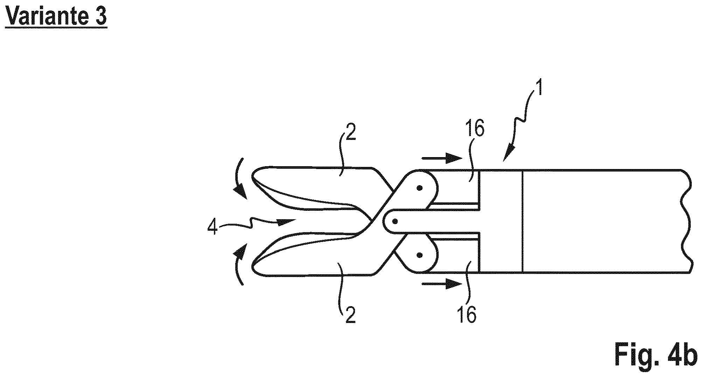

Gemäß der Fig. 4 ist der Instrumentenkopf 1 bzw. die Instrumentenbranchen 2 in die Hülse 12 zurückgezogen bzw. diese vorgeschoben, wodurch die Instrumentenbranchen 2 gegen ihre Eigenelastizität unter Verengen des Schneidspalts 4 zusammengedrückt werden. Um diesen Zusammendrückvorgang zu präzisieren, kann die Hülse 12 an ihrem distalen Ende mit einem Innenring oder Wulst 14 ausgeformt sein, der mit dem Instrumentenkopf 1 bzw. den Instrumentenbranchen 2 in Gleiteingriff mit Führungsqualität kommt / steht.

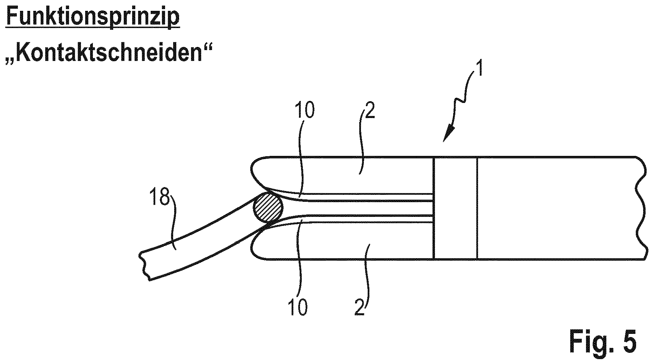

In Fig. 5 ist eine dritte Variante einer Branchenkonstruktion gezeigt. In diesem Fall sind die Branchen 2 per se vorzugsweise starr ausgebildet, jedoch schwenkbar am Instrumentenkopf 1 (scherenartig) gelagert. Ferner ist eine Betätigungs- oder Einstelleinrichtung in Form einer Zug-/Schubstange 16 vorgesehen, die an die Branchen 2 angelenkt ist, um eine Axialbewegung der Stange 16 in eine Schwenkbewegung der Branchen 2 zu transformieren. Auf diese Weise kann der Schneidspalt zwischen den Branchen 2 justiert werden.

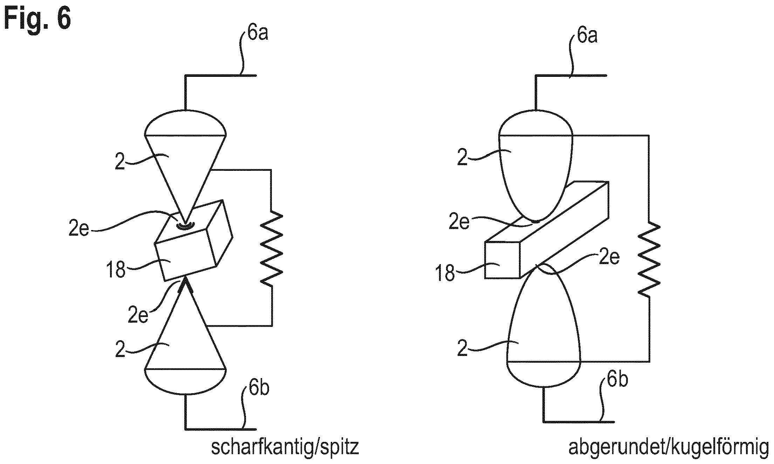

Gemäß der Fig. 5 sind dabei die Branchen 2 in einer maximalen Spaltweitenposition dargestellt, wohingegen die Fig. 6 das Instrument mit Instrumentenbranchen 2 zeigt, die mittels der Einstelleinrichtung zusammengeklappt sind, um eine minimale Spaltweite zu definieren.

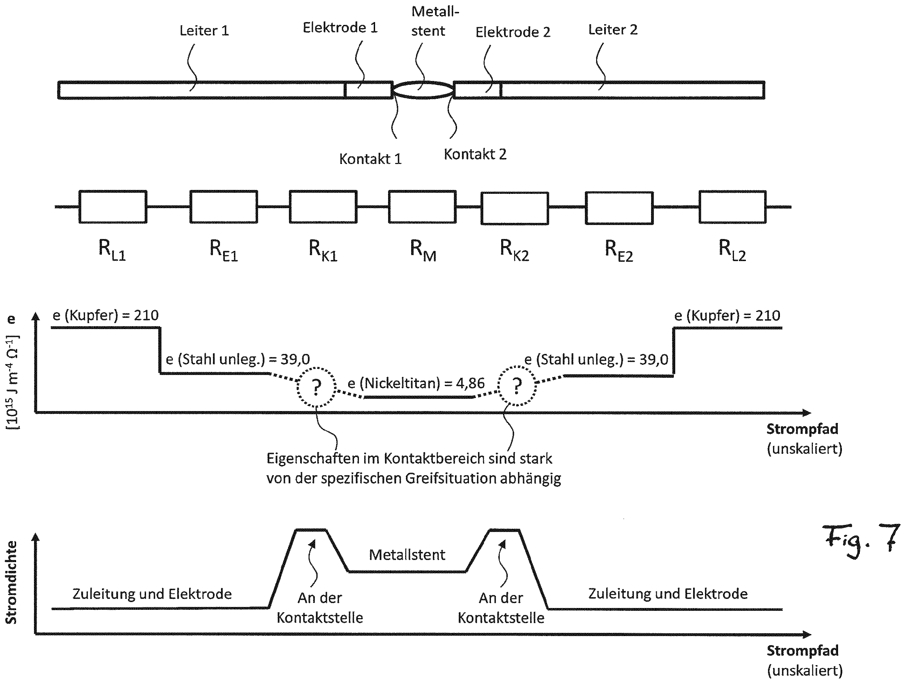

In der Fig. 7 ist das Funktionsprinzip des gleichstrombetriebenen Implantat-Schneidinstruments der Bipolarbauart gemäß der Erfindung dargestellt.

Demnach sind die Instrumentenbranchen 2 grundsätzlich derart beabstandet oder beabstandbar, dass der dazwischen sich längsausbildende Schneidspalt 4 eine Spaltweite hat, die ein Einführen eines Implantats oder Implantatabschnitts 18 (Stentdraht) in den Spalt 4 unter in Anlage kommen mit beiden sich gegenüberliegenden Längs - Branchen 2 bzw. Elektroden 10 ermöglicht / gewährleistet. Vorzugsweise sind die Instrumentenbranchen 2 hierfür zumindest im Bereich ihrer distalen (freien) Endabschnitte bananenförmig / konvex gerundet, um an den sich einander zugewandten Branchenseiten zumindest längsabschnittsweise eine konvexe Längskurve zu bilden. Dadurch verjüngt dich der Schneidspalt 4 nicht (unbedingt) linear sondern entlang einer Kurve. Es liegt dabei auf der Hand, dass die Elektroden 10 diesem Kurvenverlauf angepasst sind.

Wird nunmehr ein metallisches Implantat oder ein Implantatabschnitt 18 in den Spalt 4 eingeführt, kommt das Implantatmaterial in der Regel bereits am distalen Endabschnitt der Instrumentenbranchen 2 mit den jeweiligen Elektroden 10 in Anlage und schließt diese kurz, wodurch infolge des angelegten Leistungsstroms das zwischen den Elektroden 10 befindliche Implantatmaterial aufgeheizt und geschmolzen wird.

Wird nunmehr der Instrumentenkopf 1 vorwärts bewegt, gleitet das Implantat 18 immer tiefer in den sich verjüngenden Schneidspalt 4 hinein, wobei die Elektroden 10 das aufgeschmolzene Implantatmaterial vorzugsweise auch zerteilen.

Für den beschriebenen Zerteilungsvorgang sind demnach u. a. die folgenden Parameter von Bedeutung:

- Die Elektroden 10 bzw. die Instrumentenbranchen 2 sollten vorzugsweise eine Form haben, welche den Zerteilungsprozess begünstigt,
- Der Kontaktwiderstand zwischen den Elektroden 10 und dem Implantat 18 sollte möglichst hoch sein, um das Implantatmaterial im Kontaktbereich (d.h. von Außen nach Innen) sicher aufzuschmelzen, dabei jedoch weiter abgelegene Implantatbereiche möglichst ungeheizt zu belassen und
- Der Energieeintrag in das Implantatmaterial sollte so erfolgen, dass ein Wärmeabfluss in das umliegende Patentengewebe auch bei Fehlen zusätzlicher Schutzmaßnahmen möglichst gering bleibt.

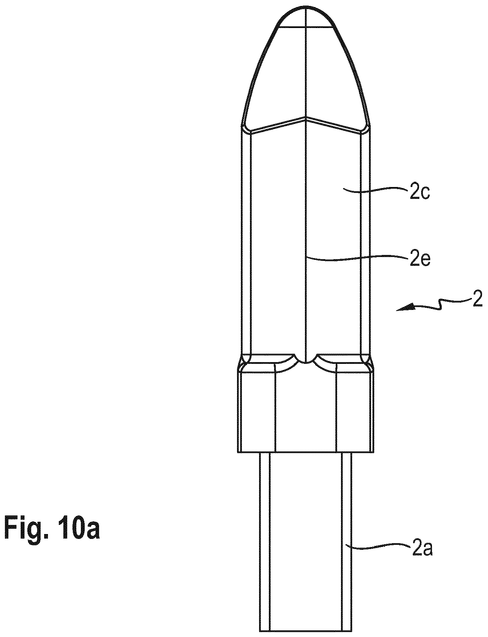

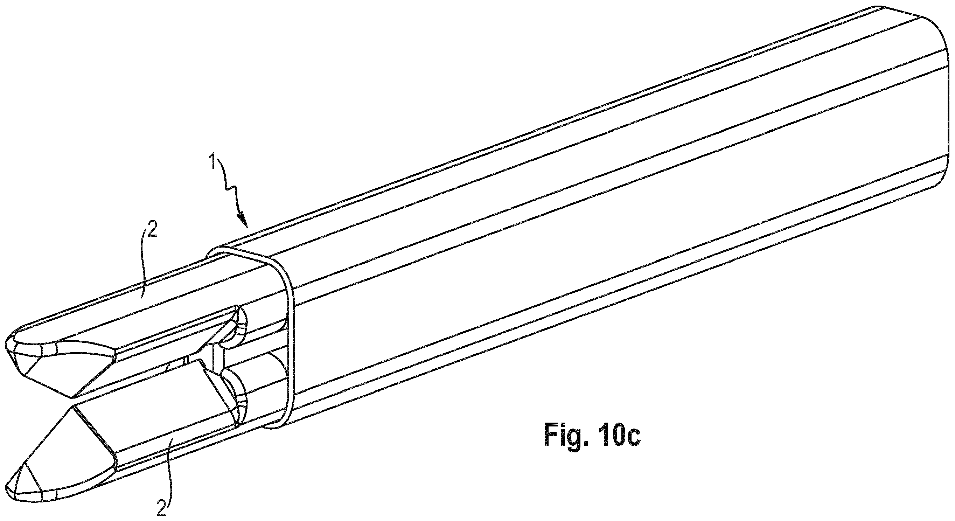

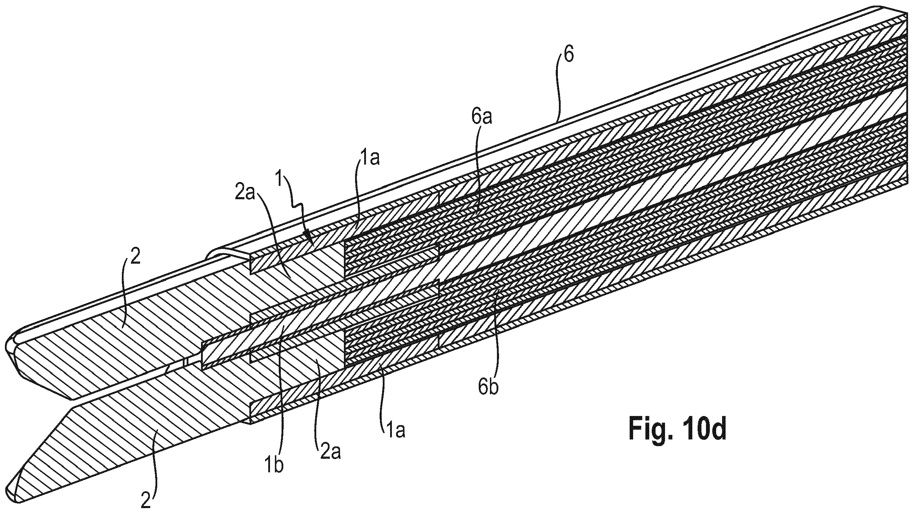

Aus diesem Grund besitzen die Instrumentenbranchen 2 und/oder zumindest die Elektroden 10 gemäß einem bevorzugten Ausführungsbeispiel der Erfindung eine Art Klingenform mit einer schmalen (scharfen) Längskante auf der jeweils sich zugewandten Längsseite jeder Branche 2. Durch diese Form wird ein (im wesentlichen eindimensionaler) punktartiger Kontakt mit dem Implantat erreicht, wodurch an dieser Stelle der Kontaktwiderstand und damit die Stromdichte und die dadurch erzeugte Materialerwärmung bei Gleichstrombeaufschlagung besonders hoch wird. Eine bevorzugte Konstruktion für eine erfindungsgemäße Instrumentenbranche 2 und/oder Elektrode ist in den Figuren 10a ff. dargestellt.

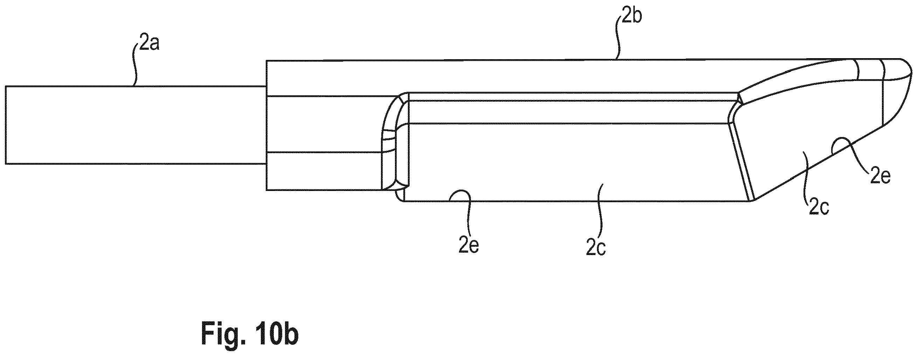

Gemäß Fig. 10a und 10b ist im bevorzugten Ausführungsbeispiel die Instrumentenbranche 2 und die Elektrode 10 (stoff-) einstückig ausgebildet, d.h. die Instrumentenbranche 2 bildet auch gleichzeitig die Elektrode 10. Demnach ist die Instrumentenbranche 2 aus einem hitzebeständigen Material, vorzugsweise Wolfram oder einem niedrig legierten Stahl gefertigt.

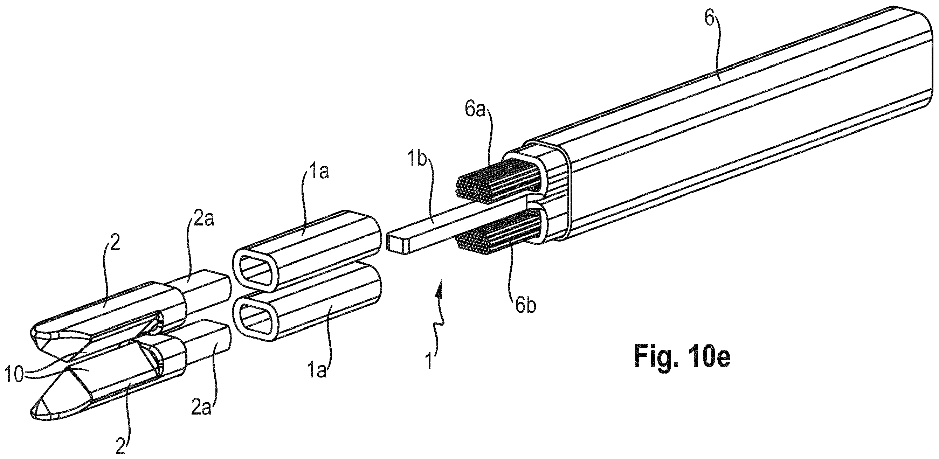

Jede Branche 2 ist an ihrem proximalen Endabschnitt zu einer Art hohler Steckstift 2a ausgeformt, der gemäß der Fig. 10e in eine entsprechende Aufnahmebuchse / Aufnahmehülse 1 a auf Seiten des Instrumentenkopfs 1 eingesteckt ist. Demnach sind für die beiden Branchen 2 zwei Aufnahmehülsen 1 a vorgesehen, die gemäß der Fig. 10e durch ein Isolier-/Diastanzstück 1 b des Instrumentenkopfs 1 voneinander getrennt sind.

Der distale Abschnitt jeder Branche 2 ist zu einer Klingenform ausgebildet (geschliffen/gefräst) mit einem teilkreisförmigen Klingenrücken 2b und einer in Längsrichtung bananenförmig geschwungenen Klingenkante 2c. Alternativ kann die Klingenform auch so gestaltet sein, dass zwei stumpfwinklig zueinander stehende (gerade) Längsabschnitte vorgesehen sind, wodurch unter Ausbildung eines singulären Knicks die Bananenform angenähert ist. Auf diese Weise entsteht an der einen Längsseite jeder Branche 2 eine schmale (scharfe) Längskante / Schneidkante 2e, die sich bogenförmig in Branchenlängsrichtung (entspricht der Instrumentenlängsrichtung) erstreckt.

Aus dem Instrumentenschaft 6 ragen an dessen distalem Ende zwei Leiterbündel 6a, 6b jeweils bestehend aus einer Vielzahl von einzelnen Litzen vor, wobei die Leiterbündel 6a, 6b ebenfalls in die Aufnahmehülsen 1a eingesteckt und vercrimpt sind. Auf diese Weise erhält jede Instrumentenbranche 2 bzw. Elektrode 10 elektrischen Kontakt mit jeweils einem Leiterbündel 6a, 6b, die wiederum an den Gleichstromgenerator angeschlossen sind.

Durch die vorstehend beschriebene Instrumentenkonstruktion können die Leiterbündel 6a, 6b trotz geringen max. Schaftaußendurchmessers eine vergleichsweise große Leiterdicke erhalten, um einen ausreichenden elektrischen Strom bei vergleichsweise niederer Stromdichte zu leiten (ohne sich zu erwärmen). Der Übergang vom jeweiligen Leiterbündel 6a, 6b vorzugsweise aus Kupfer auf die zugehörige Branche 2 vorzugsweise aus Wolfram/Stahl erfolgt über die Klemmhülse 1a und damit ohne große Verluste. Jede Branche 2 bildet dabei die eine Schneidkante 2e aus, die gleichzeitig auch die Kontaktlinie mit dem Implantat 18 definiert und die somit einen hohen Kontaktwiderstand (und damit hohe Stromdichte) generiert. Gleichzeitig dient die Schneidkante 2e vorzugsweise zum mechanischen Zerteilen des elektrisch aufgeschmolzenen Implantatmaterials.

Um den richtigen Kontaktschluss mit dem Implantat 18 sicher zu stellen, hat das chirurgische Instrument eine Kontaktqualitäts - Erkennungsfunktion, wie sie nachfolgend anhand der Fig. 6 - 9 näher beschrieben wird.

In der Fig. 6 ist der Kontaktgriff zwischen Instrument und Implantat funktionell dargestellt. Idealer Weise wird ein elektrischer Strom über die Kupferleitungen 6a, 6b im Instrumentenschaft 6 zu den Instrumentenbranchen 2 geführt und von dort über den Punktkontakt in den Implantatabschnitt zwischen den Branchen eingeleitet. Wie vorstehend ausgeführt wurde, wird dadurch ein hoher Kontaktwiderstand erreicht. Es kann nunmehr aber die Situation eintreten, dass das Implantat 18 nicht exakt zwischen den Kontaktkanten 2e der Instrumentenbranchen 2 bzw. Elektroden eingeklemmt wird sondern großflächig an der Elektrode jeder Branche 2 anliegt. Dadurch würde der Kontaktwiderstand zwischen der Elektrode und dem Implantat deutlich verringert werden (Stromdichte nimmt ab), sodass viel mehr Strom in den Leitern 6a, 6b geführt werden müsste, um ein Aufschmelzen des Implantatmaterials zu erreichen. Dies gilt es zu vermeiden, um ein Aufheizen des gesamten Implantats zu verhindern.

In der Fig. 7 ist der Verlauf der Materialeigenschaften im Strompfad des Implantatschneidinstruments gezeigt.

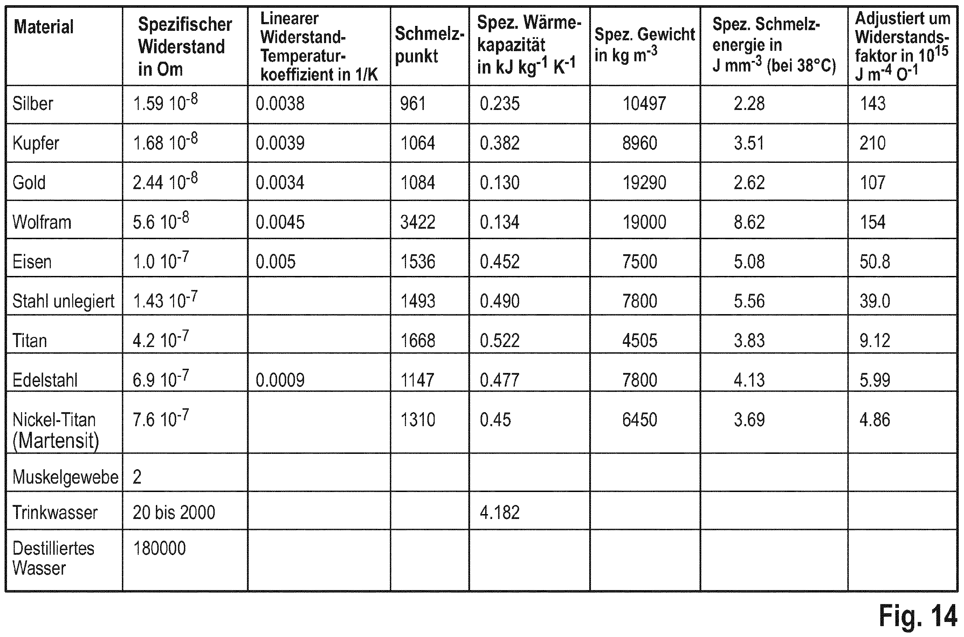

Zum Verständnis der erfindungsgemäßen Materialauswahl/-eigenschaft soll der Begriff der ‚Widerstands-adjustierten spezifischen Schmelzenergie' e eingeführt werden, wie diese in der Fig. 14 für einige ausgewählte Materialien angegeben ist. Diese Maßzahl beschreibt, wie leicht ein spezifisches Volumen (z.B. 1mm$^3$) eines leitenden Materials (z.B. ein bestimmtes Metall) durch einen Stromfluss aufgeschmolzen werden kann. Diese Maßzahl berechnet sich gemäß folgender Formel:

$$e = c \cdot V \cdot \rho \cdot (T_S - T_0) \cdot \frac{1}{r}$$

mit folgenden Parametern:

e      widerstands-adjustierte spezifische Schmelzenergie
c      spezifische Wärmekapazität
$V$      Materialvolumen
$\rho$      Massendichte
$T_S$      Schmelztemperatur
$T_0$      Temperatur vor Energieeintrag
r      Spezifischer elektrischer Widerstand

Beispielhaft ist die Situation gemäß Fig. 14 für folgende Materialauswahl aufgezeigt:

Leiter: Kupfer
Elektrode: unlegierter Stahl
Metallstent: Nickeltitan

Die widerstandsadjustierte spezifische Schmelzenergie e ist für diese Materialien jeweils ungefähr (angegeben in $10^{15}$ J m$^{-4}$ $\Omega^{-1}$):

Kupfer: 210
unlegierter Stahl: 39
Nickeltitan: 4,86

Dies bedeutet, dass sich bei identischem Stromfluss mit gleicher Stromdichte, Nickeltitan im Verhältnis 39 zu 4,86 (ca. 8x) schneller aufschmelzen lässt als unlegierter Stahl, und sich Nickeltitan entsprechend im Verhältnis 210 zu 4,86 (ca. 43x) schneller aufschmelzen lässt als Kupfer. Dies setzt gleiche Stromdichte im Material voraus. Dies bedeutet ferner, dass es durch Materialauswahl anhand der Widerstands-adjustierten spezifischen Schmelzenergie technisch ermöglicht wird, einen elektrischen Gleichstrom, der sowohl durch den Leiter, die Elektrode und das Stentmaterial fließt, so zu dosieren, dass er selektiv das Stentmaterial aufschmilzt, während die Elektroden und der Leiter die Schmelztemperatur während des Strompulses nicht erreichen. Vorzugsweise sind die Materialien so gewählt, dass die Widerstands-adjustierte spezifische Schmelzenergie der Materialien, die nicht aufgeschmolzen werden sollen (insbesondere Leitermaterial und Elektrodenmaterial) mindestens das Zweifache der Widerstands-adjustierten spezifischen Schmelzenergie derjenigen Materialien ist, die aufgeschmolzen werden sollen (insbesondere das Material des zu schneidenden Implantats).

Des Weiteren und basierend hierauf ist es erfindungsgemäß vorgesehen, die Stromdichte derart zu steuern, dass die Stromdichte im aufzuschmelzenden Zielmetall höher ist als in den Leitungen und Elektroden des Instruments. Dies wird durch die erfindungsgemäße Ausgestaltung der Elektroden gelöst, wodurch eine punktförmige

Kontaktfläche zwischen Elektrode und dem zu schneidenden Metall erzeugt wird, die zu einer hohen Stromdichte führt. Dadurch wird das Aufschmelzen des Zielmaterials im Vergleich zum Elektroden- und Leitungsmaterial zusätzlich beschleunigt. Die Kontaktqualität zwischen Elektrode und dem aufzuschmelzenden Metall ist stark von der jeweiligen Greifsituation abhängig. Eine optimale Kontaktqualität wird durch die erfindungsgemäße Ausgestaltung der Elektroden begünstigt. Zudem ist es sinnvoll, eine elektrische Überprüfung der Kontaktqualität gemäß der vorliegenden Erfindung durchzuführen.

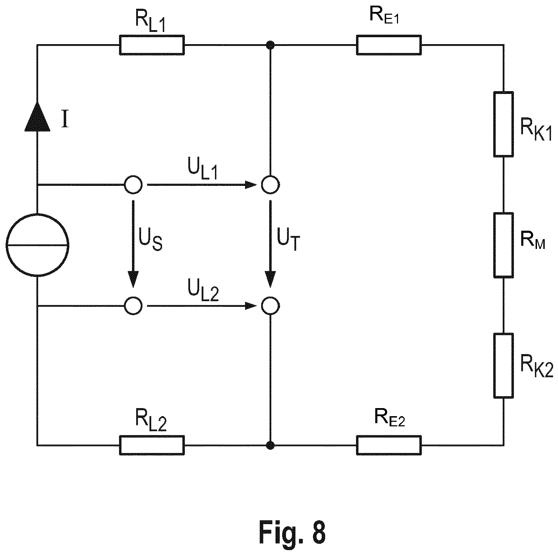

Der Widerstandsverlauf längs des elektrischen Pfads ist in Fig. 7 unten angezeichnet. Wie diesem deutlich zu entnehmen ist, stellen die beiden Kontaktstellen Widerstands-Spitzen dar, wohingegen der Strom in den übrigen Pfadabschnitten nahezu verlustfrei geleitet wird. Um nunmehr die Kontaktqualität zu bestimmen, sieht die Erfindung die Ausführung einer Testsequenz vor, die mit jedem neuen Eingriff automatisch oder auf manuellen Befehl hin wahlweise ausgeführt wird und die in der Fig. 8 schematisch dargestellt ist.

Demzufolge bilden die einzelnen Leitungsabschnitte des elektrischen Pfads individuell bestimmbare Widerstände, von denen lediglich die Kontaktwiderstände $R_{K1, 2}$ zunächst von Interesse sind. Der Leitungspfad setzt sich dabei zusammen aus den Leiterbündeln 6a, 6b im Instrumentenschaft 6, den Branchen 2, den Kontaktstellen sowie dem Implantat 18. Um die Kontaktwiderstände ausschließlich zu bestimmen, wird beispielsweise ein geringer Teststrom unterhalb des späteren Schneidstroms an den elektrischen Pfad angelegt und eine Referenzspannung unmittelbar am distalen Ende der Leiterbündel 6a, 6b abgegriffen. Aus der Differenz zwischen der angelegten und abgegriffenen Spannung kann der Leiterbündel-Widerstand errechnet werden. Ferner lässt sich hierdurch der Kontaktwiderstand $R_{K1, 2}$ bestimmen. Je nach Höhe des Kontaktwiderstands $R_{K1, 2}$ ist die Kontaktqualitäts - Erkennungsfunktion in der Lage, ein Warnsignal für eine fehlerhafte Griffposition zu geben und/oder die Zufuhr eines Schneidstroms zu blockieren und/oder die Höhe des Schneidstroms unterhalb der Belastungsgrenze der Leiterbündel 6a, 6b zu korrigieren.

An dieser Stelle sei darauf hingewiesen, dass anstelle des kleinen Teststroms auch der Schneid-/Leistungsstrom selbst zu Testzwecken verwendbar ist, wobei in diesem Fall der Schneidstrom für eine nur kurze Zeit angelegt werden darf, um den Energieeintrag in das Implantat kleiner zu halten als der Energieeintrag, der für ein Aufschmelzen des Implantatmaterials erforderlich wäre.

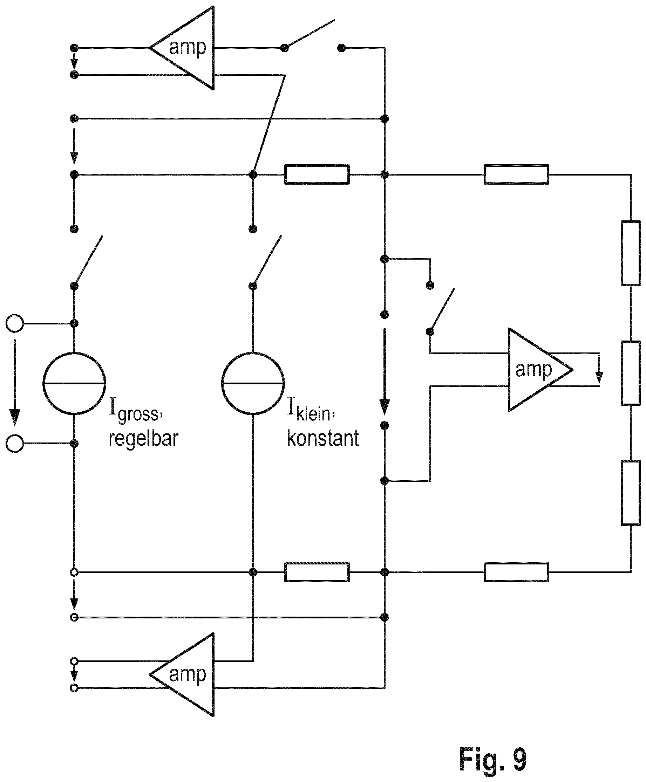

Für das nachfolgende Scheiden gemäß der Fig. 9 wird eine zur Umsetzung des Funktionsprinzips gemäß der Fig. 8 geeignete Schaltung (siehe Fig. 9) betätigt, um den zunächst anliegenden Teststrom/Leistungsstromsequenz abzuschalten und durch einen Leistungsstrom (Schneidstrom) zu ersetzen, der, wie dargestellt ist, zumindest in Abhängigkeit des bestimmten Kontaktwiderstands $R_{K1, 2}$ regelbar ist. Des Weiteren können Faktoren wie die Schneidtemperatur, die Spannungshöhe, die Temperatur im Leiterbündel, etc. in die Einstellungsprozedur des Schneidstroms einfließen. Ggf. kann eine "Not aus" - Funktion vorgesehen sein, die beispielsweise im Fall eines Kontaktabbruchs an den Elektroden die Stromzufuhr abschaltet, um Lichtbögen zu vermeiden.

**Wirkungsweise "Metalltrennen mit Gleichstrom" gemäß der vorliegenden Erfindung**

**Grundsätzliche energetische Betrachtung**

Das Kristallgitter eines Metalls hat eine kennzeichnende thermische Energie, die sich in Schwingungen der Atome des Gitters äußert. Thermische Energie ist damit eine kinetische Energie.

Die thermische Energie eines Stoffs ist von der Temperatur T, der Masse m und einer Materialkonstante c (spezifische Wärmekapazität) abhängig und lässt sich durch die folgende Formel ausdrücken:

$$E_{th} = c \cdot m \cdot T$$

Ein elektrischer Gleichstrom, der durch ein Metallgitter fließt, führt aufgrund von Kollisionen bzw. Wechselwirkungen zwischen den fließenden Ladungsträgern und den Gitteratomen zu einer Anregung der Gitteratome, folglich zu einer Erhöhung von deren kinetischer Energie und demzufolge zu einer Erhöhung der thermischen Energie (Erwärmung). Die elektrische Energie eines Gleichstromimpulses hängt von der elektrischen Leistung P und der Pulsdauer $\Delta t$ ab:

$$E_{el} = P \cdot \Delta t$$

Sowohl die thermische als auch die elektrische Energie wird üblicherweise mit den Einheiten Wattsekunden (Ws) oder Joule (J) angegeben.

Bei Durchfließen eines Metallstücks wird die elektrische Energie vollständig in thermische Energie umgesetzt, die zu einer thermischen Energiedifferenz zwischen vorher (t0) und danach (t1) führt. Während der Dauer des Impulses führt die Temperaturdifferenz zwischen dem stromdurchflossenen Abschnitt des Metallgitters und benachbarten Strukturen zu einem Abfluss thermischer Energie $E_{Diss}$ in die Umgebung. Dieser Abfluss ist von verschiedenen Materialkonstanten abhängig, die in der Variable k gebündelt und nicht näher spezifiziert werden, sowie von dem Integral der Temperaturdifferenz über die Zeit Δt:

$$E_{Diss} = k \cdot \int_{\Delta t} \Delta T(t) \cdot dt$$

Der Abfluss der thermischen Energie in die Umgebung hat nach obigem Zusammenhang eine starke Abhängigkeit von der Impulsdauer Δt. D.h., je kürzer der Impuls, desto weniger thermische Energie fließt ab.

Die Differenz der thermischen Energie zwischen vorher (t0) und nachher (t1) lässt sich wie folgt beschreiben:

$$E_{th,t1} = E_{th,t0} + E_{el} - E_{Diss}$$

Die Temperaturerhöhung ΔT lässt sich dann wie folgt berechnen:

$$\Delta T = (T_{t1} - T_{t0}) = \frac{E_{el}}{c \cdot m} - E_{Diss}$$

Mit Einsetzen der Massendichteformel und Separierung der Materialkonstanten ergibt sich folgende Proportionalitätsbeziehung:

$$\Delta T = \frac{1}{c \cdot \rho} \cdot \frac{E_{el}}{V} - k \cdot \int_{\Delta t} \Delta T(t) \cdot dt$$

Nach obigem Zusammenhang hat man somit drei Möglichkeiten, die Temperaturerhöhung in einem Implantatsmaterial zu steigern:

a) Man erhöht die zugeführte elektrische Energie $E_{el}$
b) Man reduziert das vom Gleichstrom durchflossene Volumen V
c) Man reduziert die Impulsdauer (um den Wärmeabflusseffekt zu minimieren)

Eine Senkung der Impulsdauer gemäß Punkt c) führt gleichzeitig zu einer Reduzierung der elektrischen Energie $E_{el}$ gemäß Punkt a). Dies kann jedoch mit einer Erhöhung der elektrischen Leistung P kompensiert werden. Es ist daher zweckmäßig, die Impulsdauer Δt zu minimieren (um den Wärmeabfluss während dem Energieeintrag zu minimieren) und gleichzeitig die elektrische Leistung P zu maximieren (um trotz geringer Impulsdauer ausreichend elektrische Energie $E_{el}$ zuzuführen).

Erfindungsgemäß ist es daher vorgesehen, dass das bipolare Schneidinstrument eine möglichst starke lokale Erwärmung eines gegriffenen Metallstücks (mit dem Zielpunkt der lokalen Aufschmelzung) mit einem möglichst geringen erforderlichen Eintrag von elektrischer Energie erzeugt. Hierbei soll zwar das gegriffene Zielmetall aufgeschmolzen werden, das Instrumentenmaterial aber intakt bleiben und das umgebende Gewebe nicht beschädigt werden.

**Erwärmungsprozess, Einfluss von Materialkonstanten**

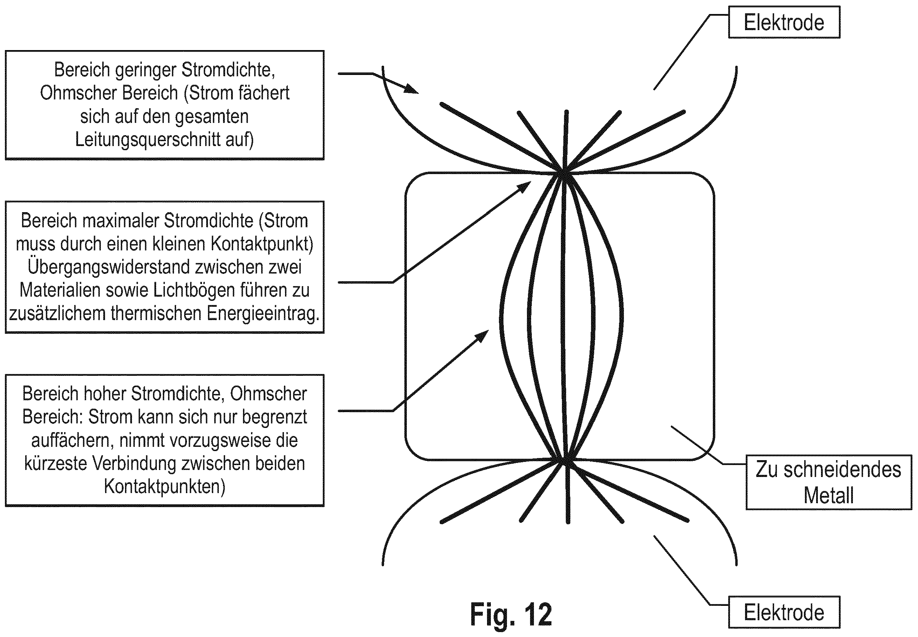

Einfluss auf den Schneideprozess haben zum einen Materialkonstanten, zum anderen die geometrischen Verhältnisse den Strompfad betreffend, die insbesondere die Stromdichte bestimmen, wie dies in der Fig. 12 dargestellt ist.

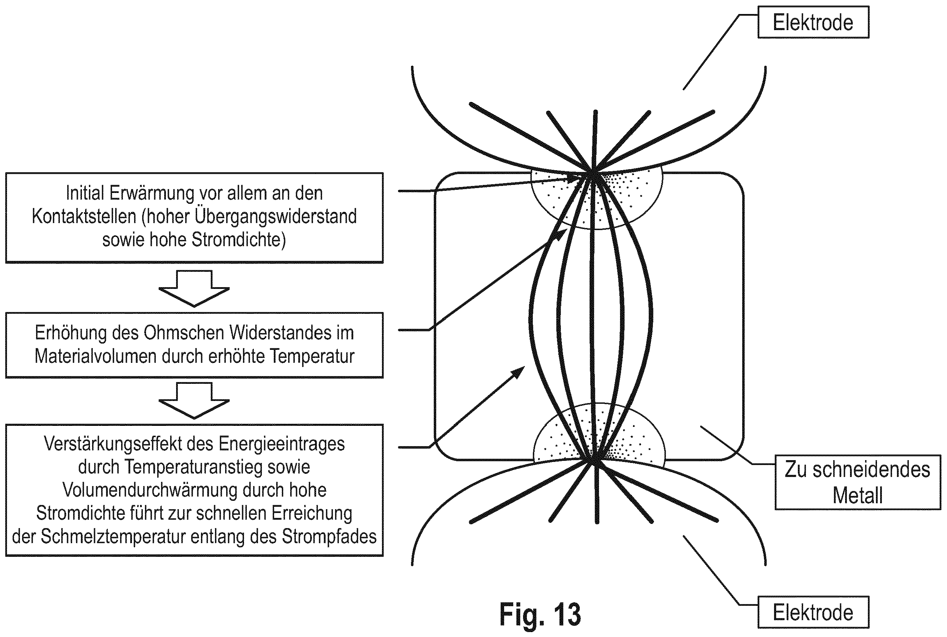

Das Aufschmelzen eines Volumenelements durch elektrischen Gleichstrom gemäß der Fig. 13 lässt sich wie folgt berechnen:

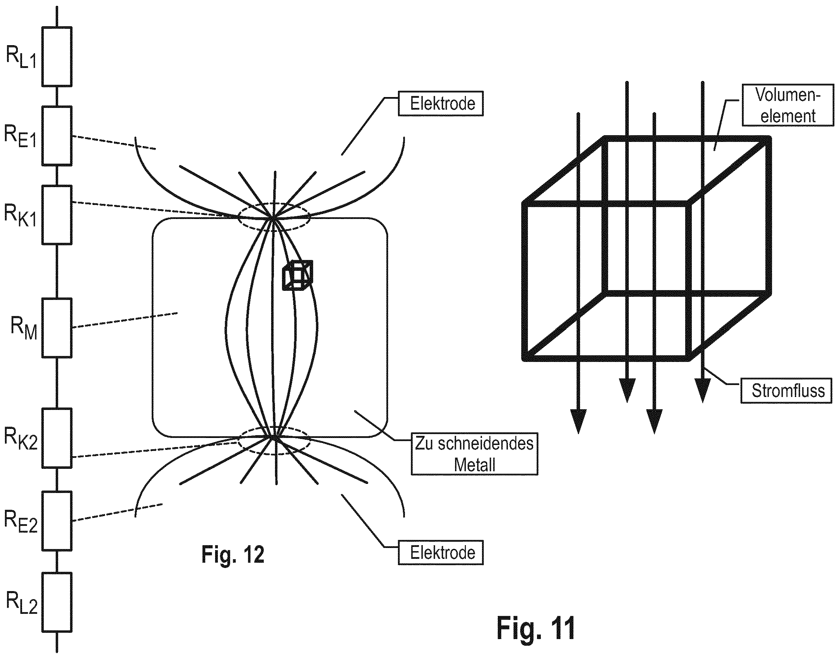

Die thermische Energie $E_{th}$, die nötig ist, um ein Volumenelement gemäß Fig. 11 eines Materials aufzuschmelzen, ist abhängig von der Anfangstemperatur $T_0$ der Schmelztemperatur $T_S$, der spezifischen Wärmekapazität c sowie dem spezifischen Gewicht (Massendichte) p:

$$E_{th} = c \cdot \rho \cdot V \cdot (T_S - T_0)$$

Für $T_0$ = 38°C (Körpertemperatur) und einem Standardvolumen von 1mm³ ergeben sich Werte für die Schmelzenergie pro mm³ gemäß der nachfolgenden Tabelle (Fig. 14). Die Bereitschaft eines Materials, elektrische Energie in thermische Energie umzusetzen, ist proportional zum ohmschen Widerstand des Materials. Um dies für den Anwendungsfall des erfindungsgemäßen Implantat - Schneidinstruments (bei dem derselbe Strom durch verschiedene Materialien fließt) zu berücksichtigen, wird die spezifische Schmelzenergie um den ohmschen Widerstand adjustiert (durch Multiplikation mit dem Kehrwert des Widerstandes). Daraus ergibt sich ein Maß, anhand dessen eine Vergleichbarkeit der Materialien hinsichtlich Ihrer Energieaufnahmeeigenschaften und Eignung zur Verwendung im Strompfad möglich ist. So eignet sich beispielsweise unlegierter Stahl als Elektrodenmaterial zum Schneiden von Nickel-Titan insbesondere sehr gut, da diese Maßzahl (die um den Widerstandsfaktor adjustierte spezifische Schmelzenergie) für unlegierten Stahl mit 39 deutlich höher ist als für Nickel-Titan mit 4,86 (letzte Spalte in Fig. 14). In anderen Worten, wenn beide Materialien mit demselben Strom durchflossen werden, schmilzt Nickel-Titan im Verhältnis 39 zu 4,86 schneller auf als unlegierter Stahl.

Zusammenfassend wird ein mit Gleichstrom betriebenes chirurgisches Implantatschneideinstrument der Bipolarbauart offenbart mit einem am distalen Ende eines Instrumentenschafts befindlichen Instrumentenkopf für ein minimal invasives Einführen des Instruments in einen Patientenkörper, wobei am Instrumentenkopf zumindest zwei sich gegenüberliegende Instrumentenbranchen vorzugsweise der Linearbauart angeordnet sind, die zwischen sich einen Schneidspalt zur dazwischen Aufnahme eines elektrisch leitenden Implantats oder Implantatsabschnitts definieren. Erfindungsgemäß sind an deren einander zugewandte Branchenlängsseiten Elektroden ausgebildet oder jeweils mit zumindest einer Elektrode bestückt, die wiederum an ihren einander zugewandten Längsseiten jeweils zu einer Schneidkante geformt sind, um einen punktförmigen körperlichen Kontakteingriff mit dem elektrisch leitenden Implantat oder Implantatsabschnitt für einen elektrischen Kurzschluss der sich gegenüberliegenden Elektroden zu bewirken.

Kontaktqualitätserkennung

Beim Aufschmelzen des Zielmetalls ist es erfindungsgemäß vorgesehen, dies mittels eines Leistungs-Gleichstroms (im Bereich von ca. 100-150 Ampere) über ein endoskopisches Instrument zu bewerkstelligen. Hierbei ist es wichtig, dass das Zielmetall des Implantats selektiv aufgeschmolzen wird, d.h. dass der Gleichstrom zwar das Stentmaterial, aber nicht das Leitungs- bzw. Elektrodenmaterial des endoskopischen Implantatschneidinstruments aufschmilzt. Eine entsprechende Dosierung (Einstellung) von Stromstärke und Impulsdauer bzw. -Form ist hierfür maßgebend.

Unter Kontaktqualität versteht man in diesem Zusammenhang die für Stromdichte und Strompfadlänge gesetzten Rahmenbedingungen, die sich bei Kontaktierung des Implantatmaterials mit dem Instrument ergeben. Einflussfaktoren können beispielsweise die konkrete Ausformung des gegriffenen/kontaktierten Implantatabschnitts sein. Ist der der gegriffene/kontaktierte Implantatabschnitt zwischen den Elektroden dünn und flächig, so ergibt sich eine geringe Stromdichte, ist er eher schmal, ergibt sich eine hohe Stromdichte. Die konkrete Kontaktierungssituation kann individuell sehr unterschiedlich ausfallen, so dass sich unterschiedliche Kontaktqualitäten ergeben. Um ein zuverlässiges Schneiden zu gewährleisten und gleichzeitig einen übermäßigen Energieeintrag zu vermeiden (der zu Verpuffungseffekten führen oder das Instrument beschädigen kann), ist es zweckmäßig, die Stromstärke und Impulsdauer bzw. -form entsprechend der Kontaktqualität einzustellen.

Die Kontaktqualität kann über eine Widerstandsmessung durchgeführt werden. Der Strompfad lässt sich gemäß Fig. 7 über ein Ersatzschaltbild mit folgenden in Serie angeordneten Ohmschen Widerständen beschreiben:

$R_{L1}$     Widerstand des Leiters 1
$R_{E1}$     Widerstand der Elektrode 1
$R_{K1}$     Kontaktwiderstand (Elektrode 1 - Stentmaterial) 1
$R_M$      Widerstand des zu schneidenden Metalls
$R_{K2}$     Kontaktwiderstand (Elektrode 2 - Stentmaterial) 2
$R_{E2}$     Widerstand der Elektrode 2
$R_{L2}$     Widerstand des Leiters 2

Die Kontaktqualitätsbestimmung erfolgt nach dem Prinzip der Vierleitermessung. Dies hat Anwendung vor dem eigentlichen Schneideprozess zur Bestimmung geeigneter Schneideparameter (Stromstärke, Impulsdauer und -form), sowie während dem Schneideprozess. Das Grundprinzip ist in Fig. 8 dargestellt.

In einer Ausführungsvariante der Kontaktqualitätsbestimmung wird vor dem Schneideprozess ein kleiner Teststrom in das Instrument derart eingeleitet, dass der Teststrom über die Elektroden durch das zu schneidende Stentmaterial fließt. Über zwei Leitungen, die mit den Elektroden elektrischen Kontakt haben, aber durch die nicht der Teststrom fließt, lässt sich der durch den Teststrom erzeugte Spannungsabfall $U_T$ zwischen den zwei Elektroden messen. Dies entspricht einer Messung der Widerstände $R_{E1}$, $R_{K1}$, $R_M$, $R_{K2}$ und $R_{E2}$ im Ersatzschaltbild nach dem Prinzip der Vierleitermessung. Der Teststrom liegt deutlich unterhalb des Leistungsstroms, vorzugsweise im Milliampere-Bereich (z.B. 20 mA). Neben der Bestimmung der Kontaktqualität kann so auch ermittelt werden, ob überhaupt ein elektrisch leitender Kontakt hergestellt wurde.

In einer weiteren Ausführungsvariante der Kontaktqualitätsbestimmung wird vor dem Schneideprozess (vorzugsweise wenn ein stabiler elektrischer Kontakt festgestellt wurde) ein Testimpuls durch das zu schneidende Implantatmaterial geschickt, der zu einer Erwärmung aber nicht zum Aufschmelzen führt. Eine Erwärmung führt zu einer Erhöhung des spezifischen Widerstandes des Materials und folglich zu einer Erhöhung der nach dem Prinzip der Vierleitermethode ermittelten Spannung $U_T$ zwischen den Elektroden. Während dieses Impulses lässt sich anhand der Anstiegskurve des elektrischen Widerstandes zwischen den Elektrode (entsprechend den Widerständen $R_{E1}$, $R_{K1}$, $R_M$, $R_{K2}$ und $R_{E2}$ im Ersatzschaltbild) rechnerisch eine Prognose des zum Aufschmelzen erforderlichen Energieeintrags ableiten. Dies kann als Grundlage für die Einstellung der Schneidparameter dienen.

Während dem Schneideprozess lässt sich aus der Überwachung der Spannung $U_T$ zwischen den Elektroden der Schneideprozess überwachen mit dem Ziel, bei Bedarf die Schneideparameter so anzupassen, dass z.B. ein absehbar zu niedrig eingestellter Parameter korrigiert wird.

Des Weiteren werden die Spannungen $U_{L1}$ und $U_{L2}$ ermittelt. Dies erfolgt sowohl mit dem Teststrom vor dem Schneideprozess als auch mit dem Leistungsstrom während dem Schneideprozess. Da sich der ohmsche Widerstand des Leitermaterials mit der Temperatur verändert, kann die Messung des Spannungsabfalls bei bekanntem Strom der ohmsche Widerstand und damit die Temperatur ermittelt werden. Dies ist insbesondere zweckmäßig, um ein Überhitzen des Instrumentenschaftes zu erkennen und durch entsprechende Einstellung der Schneideparameter bzw. Abbruch des Schneideprozesses zu vermeiden. Der Temperaturkoeffizient verschiedener leitender Materialien ist in Fig. 14 dargestellt.

Da der Teststrom vorzugsweise um ein vielfaches kleiner als der Leistungsstrom ist, ist es zweckmäßig, die Messung der Spannungen $U_T$, $U_{L1}$ und $U_{L2}$ wahlweise elektronisch verstärken zu können. Eine vereinfachte entsprechende Schaltung ist in Fig. 9 dargestellt.

**Patentansprüche**

1. Auf Gleichstrombasis arbeitendes chirurgisches Implantatschneideinstrument der Bipolarbauart mit einem Instrumentenkopf (1), an dem zumindest zwei sich gegenüberliegende Instrumentenbranchen (2) angeordnet sind, die zwischen sich einen in Instrumentenlängsrichtung sich erstreckenden Schneidspalt (4) zur dazwischen Aufnahme eines elektrisch leitenden Implantats oder Implantatsabschnitts (18) definieren, wobei die einander zugewandten Längsseiten der zumindest zwei Instrumenten - Längsbranchen (2) jeweils eine Elektrode (10) ausbilden oder jeweils mit zumindest einer Elektrode (10) bestückt sind und weiter eine Stromsteuereinrichtung vorgesehen ist, die dafür angepasst ist, die Elektroden mit einem Gleichstrom vorbestimmter oder einstellbarer Stärke zu beaufschlagen, **dadurch gekennzeichnet dass** die Elektroden (10) so geformt sind, dass sie an ihren einander zugewandten Elektrodenseiten einen punktförmigen Kontakt-Aufstand erhalten, und weiter dass die Stromsteuereinrichtung dafür angepasst ist, die Elektroden mit einem Gleichstrom in gepulster oder getakteter Weise zu beaufschlagen, derart, dass eine bei wenigstens einem Strompuls zugeführte Strommenge ausreicht, den zwischen den Elektroden kontaktierten Draht an den Kontaktstellen zwischen den Elektroden und dem Draht von aussen aufzuschmelzen.

2. Implantatschneidinstrument nach Anspruch 1, **dadurch gekennzeichnet, dass** die Pulsweite und/oder die Stromstärke über die Stromsteuereinrichtung für den Erhalt der erforderlichen Strommenge eingestellt oder einstellbar ist.

3. Implantatschneideinstrument nach Anspruch 1 oder 2, **dadurch gekennzeichnet, dass** die Elektroden (10) an ihren einander zugewandten Längsseiten jeweils zu einer längs sich erstreckenden Schneidkante (2e), einer längs sich erstreckenden konvexen Auswölbung oder einer Ausrundung geformt sind, wodurch sich bei miteinander in Anlage kommen der beiden Elektroden der Kontakt - Aufstandspunkt ergibt, um einen punktförmigen körperlichen Kontakteingriff mit dem dazwischen einzuführenden, elektrisch leitenden Implantat oder Implantatsabschnitt (18) für einen elektrischen Kurzschluss der sich gegenüberliegenden Elektroden (2) zu bewirken.

4. Implantatschneideinstrument nach Anspruch 1, 2 oder 3, **dadurch gekennzeichnet, dass** die sich gegenüberliegende Elektroden (2), zwischen denen sich der Schneidspalt (4) in Instrumentenlängsrichtung ausbildet, in einer Zeitperiode kleiner als eine Sekunde mit Gleichstromimpulsen vorbestimmter Stromstärke von bis zu 200 Ampere steuerungstechnisch beaufschlagt werden.

5. Implantatschneideinstrument nach einem der vorstehenden Ansprüche, **dadurch gekennzeichnet, dass** die Spannung unter einem Grenzwert von 48 Volt für eine Niederspannung liegt, vorzugsweise zwischen 20 - 40 Volt.

6. Implantatschneideinstrument nach einem der vorstehenden Ansprüche, **dadurch gekennzeichnet, dass** die beiden Instrumentenbranchen (2) starr am Instrumentenkopf (1) fixiert sind, oder dass die beiden Instrumentenbranchen (2) und/oder die daran montierten Elektroden (10) federelastisch nachgebend gestaltet und/oder am Instrumentenkopf für eine selbsttätige Spaltweiteneinstellung gelagert sind, sodass Implantate mit unterschiedlichen Materialstärken in den Schneidspalt (4) für einen Elektrodenkurzschluss einführbar sind.

7. Implantatschneideinstrument nach einem der vorstehenden Ansprüche 1 - 5, **dadurch gekennzeichnet, dass** die beiden Instrumentenbranchen (2) über einen Betätigungsmechanismus beweg- oder einstellbar sind, um die Schneidspaltweite definiert zu ändern.

8. Implantatschneideinstrument nach einem der vorstehenden Ansprüche, **dadurch gekennzeichnet, dass** die beiden Elektroden (10) und/oder die beiden Instrumentenbranchen (2) im Wesentlichen V-förmig zueinander ausgerichtet sind, sodass sich der dazwischen sich ausbildende Schneidspalt (4) in Richtung proximal kontinuierlich oder in konvexer Kurvenform verengt.

9. Implantatschneideinstrument nach einem der vorstehenden Ansprüche, **dadurch gekennzeichnet, dass** eine Vorspanneinrichtung am Instrumentenkopf (1) vorgesehen ist, welche die Elektroden (10) und/oder die Instrumentenbranchen (2) mit einer bestimmten Vorspannkraft gegeneinander vorspannt, sodass ein dazwischen eingeführtes Implantatsmaterial durch den Einführvorgang selbst automatisch zusammengedrückt oder gequetscht wird.

**10.** Implantatschneideinstrument nach einem der vorstehenden Ansprüche, **dadurch gekennzeichnet, dass** eine elektrische / elektronische Kontaktqualitätserkennung vorgesehen ist, die vorzugsweise bei einer Schneidbetätigung des Instruments sowie zeitlich vor der daraus resultierenden Elektrodenbeaufschlagung mit einem Leistungsgleichstrom oder separat und unabhängig von einer Schneidbetätigung eine Kontaktqualität - Testsequenz ausführt.

**11.** Implantatschneideinstrument nach Anspruch 10, **dadurch gekennzeichnet, dass** die Testsequenz eine Beaufschlagung der Elektroden (10) mit einem Teststrom unterhalb des Leistungsgleichstroms oder eine Beaufschlagung der Elektroden (10) mit dem Leistungsgleichstrom jedoch mit einer Testimpulsdauer unterhalb der Schneidimpulsdauer vorsieht, jeweils um den Kontaktwiderstand zwischen den jeweiligen Elektroden (10) und dem Implantat / Implantatabschnitt vorzugsweise nach dem Prinzip einer Vier-Leiter-Messung zu ermitteln.

**12.** Implantatschneideinstrument nach Anspruch 11, **dadurch gekennzeichnet, dass** die elektrische / elektronische Kontaktqualitätskennung im Fall des Unterschreitens eines Grenzwerts ein Warnsignal ausgibt und/oder die nachfolgende Leistungsgleichstrom - Beaufschlagung blockiert **und/oder** die elektrische / elektronische Kontaktqualitätserkennung bei Erreichen oder Überschreiten des Grenzwerts ein Akzeptanzsignal ausgibt und/oder die nachfolgende Leistungsgleichstrom - Beaufschlagung zulässt.

**13.** Implantatschneideinstrument nach einem der vorstehenden Ansprüche 11 und 12, **dadurch gekennzeichnet, dass** die elektrische / elektronische Kontaktqualitätskennung die Höhe des zugeführten Leitungsgleichstroms für den Implantatschneidvorgang entsprechend dem Ermittlungsergebnis bezüglich des Kontaktwiderstands anpasst.

**14.** Implantatschneideinstrument nach einem der vorstehenden Ansprüche 10 - 13, **dadurch gekennzeichnet, dass** die elektrische / elektronische Kontaktqualitätskennung in der Testsequenz nachprüft, ob der zum Aufschmelzen der Implantats oder Implantatabschnitts erforderliche Leistungsgleichstrom eine Instrumentenerwärmung unterhalb der thermischen Belastbarkeit der Zuführleitungen und/oder der Elektroden bewirkt.

**15.** Implantatschneideinstrument nach einem der vorstehenden Ansprüche, das auf Gleichstrombasis arbeitet, mit einem Instrumentenkopf (1), an dem zumindest zwei sich gegenüberliegende Instrumenten - Längsbranchen (2) angeordnet sind, die zwischen sich einen in Instrumentenlängsrichtung sich erstreckenden Schneidspalt (4) definieren, der für eine dazwischen Aufnahme eines elektrisch leitenden Implantats oder Implantatsabschnitts (18) angepasst ist, **dadurch gekennzeichnet, dass** die Instrumentenbranchen (2) oder Elektroden (10) aus einem hitzebeständigen Material, , gefertigt sind, dessen widerstands-adjustierte spezifische Schmelzenergie größer als $5.99 \cdot 10^{15}$ J m$^{-4}$ $\Omega^{-1}$ ist.

**Claims**

**1.** Surgical implant-cutting instrument of bipolar type, operating on direct current and having an instrument head (1) on which at least two mutually opposing instrument branches (2) are arranged which between them define a cutting gap (4) extending in the longitudinal direction of the instrument, for receiving therebetween an electrically conductive implant or implant section (18), wherein
the mutually facing longitudinal sides of the at least two instrument-longitudinal branches (2) each form an electrode (10) or are each equipped with at least one electrode (10), and
furthermore, a current control device is provided which is suited to apply to the electrodes a direct current of predetermined or adjustable strength, **characterized in that** the electrodes (10) are shaped so that they achieve at their mutually facing electrode sides a punctiform contact, and furthermore **in that** the current control device is suited to apply to the electrodes a direct current in a pulsed or timed way such that a quantity of current in at least one current pulse is sufficient to melt, from the outside, the wire in contact between the electrodes, at the contact locations between the electrodes and the wire.

**2.** Implant-cutting instrument according to claim 1, **characterized in that** the pulse width and/or the current strength is set or is settable via the current control device for obtaining the required quantity of current.

**3.** Implant-cutting instrument according to claim 1 or 2, **characterized in that** the electrodes (10) are each shaped at their mutually facing longitudinal sides to form a longitudinally extending cutting edge (2e), a longitudinally extending convex bulging or a rounding, whereby the contact point arises when the two electrodes are brought together, in order to effect a punctiform physical contact engagement with the electrically conductive implant or implant section (18) to be introduced therebetween, for an electrical short circuit of the mutually opposing electrodes (2).

**4.** Implant-cutting instrument according to claim 1, 2 or 3, **characterized in that** DC current impulses of predetermined current strengths of up to 200 amperes are applied in a time period smaller than one second and by means of control technology, to the mutually opposing electrodes (2) between which the cutting gap (4) is formed in the longitudinal direction of the instrument.

**5.** Implant-cutting instrument according to one of the above claims, **characterized in that** the voltage lies below a limit of 48 Volts for a low voltage, preferably between 20 - 40 Volts.

**6.** Implant-cutting instrument according to one of the above claims, **characterized in that** the two instrument branches (2) are rigidly fixed to the instrument head (1), or **in that** the two instrument branches (2) and/or the electrodes (10) mounted to them are configured springily resiliently and/or are mounted at the instrument head for an automatic setting of gap width such that implants of different material thicknesses can be introduced into the cutting gap (4) for a short-circuit of the electrodes.

**7.** Implant-cutting instrument according to one of the above claims 1 - 5, **characterized in that** the two instrument branches (2) are movable or settable via an actuating mechanism in order to change the cutting gap width in a defined way.

**8.** Implant-cutting instrument according to one of the above claims, **characterized in that** the two electrodes (10) and/or the two instrument branches (2) are oriented with respect to each other to be essentially V-shaped so that the cutting gap (4) forming therebetween narrows in the proximal direction, continuously or in a convex curve shape.

**9.** Implant-cutting instrument according to one of the above claims, **characterized in that** a biasing device is provided at the instrument head (1), which biases the electrodes (10) and/or the instrument branches (2) against each other with a predetermined biasing force so that an implant material introduced therebetween is automatically compressed or crushed by the insertion process itself.

**10.** Implant-cutting instrument according to one of the above claims, **characterized in that** an electric/electronic contact quality detection device is provided which performs a contact-quality detection test sequence, preferably in the event of a cutting operation of the instrument and chronologically before the resulting application of a DC power current to the electrodes, or separately and independently from a cutting operation.

**11.** Implant-cutting instrument according to claim 10, **characterized in that** the test sequence provides an application to the electrodes (10) of a test current below the DC power current, or an application to the electrodes (10) of the DC power current but with a test impulse duration below the cutting impulse duration, in each case in order to determine the contact resistance between the respective electrodes (10) and the implant/implant section, preferably according to the principle of four-conductor-measurement.

**12.** Implant-cutting instrument according to claim 11, **characterized in that,** in the case of falling below a threshold value, the electric/electronic contact quality detection device outputs a warning signal and/or blocks the subsequent application of DC power current **and/or,** in the case of reaching or exceeding the threshold value, the electric/electronic contact quality detection device outputs an acceptance signal and/or permits the subsequent application of DC power current.

**13.** Implant-cutting instrument according to either one of the above claims 11 and 12, **characterized in that** the electric/electronic contact quality detection device adjusts the amount of the supplied DC power current for the implant cutting process correspondingly to the determination result relative to the contact resistance.

**14.** Implant-cutting instrument according to one of the above claims 10 - 13, **characterized in that** the electric/electronic contact quality detection device verifies in the test sequence whether the DC power current required for the melting of the implant or implant section brings about a heating of the instrument below the thermal load capacity of the supply lines and/or of the electrodes.

**15.** Implant-cutting instrument according to one of the above claims, which operates on direct current and has an instrument head (1) on which at least two mutually opposing instrument-longitudinal branches (2) are arranged which between them define a cutting gap (4) extending in the longitudinal direction of the instrument, which cutting gap (4) is suited for receiving therebetween an electrically conductive implant or implant section (18), **characterized in that** the instrument branches (2) or electrodes (10) are made from a heat resistant material whose resistance-adjusted specific melting energy is larger than $5.99 \, 10^{15} \, J \, m^{-4} \, \Omega^{-1}$.

**Revendications**

**1.** Instrument de découpe d'implant chirurgical travaillant en courant continu du type bipolaire, avec une tête d'instrument (1), au niveau de laquelle au moins deux branches d'instrument (2) se faisant face sont disposées, lesquelles définissent entre elles une fente de découpe (4) s'étendant dans le sens longitudinal d'instrument, servant à recevoir de manière intercalée un implant ou un tronçon d'implant (18) électroconducteur, dans lequel les côtés longitudinaux tournés les uns vers les autres des deux branches longitudinales d'instrument (2) ou plus réalisent respectivement une électrode (10) ou sont équipés respectivement d'au moins une électrode (10), et

dans lequel par ailleurs un dispositif de commande de courant est prévu, qui est adapté pour soumettre les électrodes à l'action d'un courant continu présentant une intensité prédéterminée ou pouvant être réglée,

**caractérisé en ce que** les électrodes (10) sont formées de telle sorte qu'elles reçoivent, au niveau de leurs côtés d'électrode tournés les uns vers les autres, un support de contact point par point, et **en ce que** par ailleurs le dispositif de commande de courant est adapté afin de soumettre les électrodes à l'action d'un courant continu par impulsions ou de manière cadencée de telle manière qu'une quantité de courant amenée lors d'au moins une impulsion de courant suffit pour faire fondre le fil mis en contact entre les électrodes au niveau des emplacements de contact entre les électrodes et le fil depuis l'extérieur.

2. Instrument de découpe d'implant selon la revendication 1, **caractérisé en ce que** la largeur d'impulsion et/ou l'intensité de courant sont réglées ou peuvent être réglées par l'intermédiaire du dispositif de commande de courant pour l'obtention de la quantité de courant requise.

3. Instrument de découpe d'implant selon la revendication 1 ou 2, **caractérisé en ce que** les électrodes (10) sont formées au niveau de leurs côtés longitudinaux tournés les uns vers les autres respectivement en une arête de découpe (2e) s'étendant en longueur, en un bombement convexe s'étendant dans la longueur ou en un arrondi, ce qui permet de donner lieu au point de support de contact lorsque les deux électrodes viennent en appui entre elles afin de provoquer une prise de contact physique point par point avec l'implant ou le tronçon d'implant électroconducteur (18) à introduire de manière intercalée, pour un court-circuit électrique des électrodes (2) se faisant face.

4. Instrument de découpe d'implant selon la revendication 1, 2 ou 3, **caractérisé en ce que** les électrodes (2) se faisant face, entre lesquelles la fente de découpe (4) est réalisée dans le sens longitudinal de l'instrument, sont soumises par une technique de commande, lors d'une période de temps inférieure à une seconde, à des impulsions de courant continu présentant une intensité de courant prédéterminée pouvant aller jusqu'à 200 ampères.

5. Instrument de découpe d'implant selon l'une quelconque des revendications précédentes, **caractérisé en ce que** la tension se situe sous une valeur limite de 48 volts pour une tension basse, est comprise de préférence entre 20 - 40 volts.

6. Instrument de découpe d'implant selon l'une quelconque des revendications précédentes, **caractérisé en ce que** les deux branches d'instrument (2) sont fixées de manière rigide au niveau de la tête d'instrument (1), ou **en ce que** les deux branches d'instrument (2) et/ou les électrodes (10) montées au niveau de ces dernières sont configurées de manière flexible et élastique comme un ressort et/ou sont logées au niveau de la tête d'instrument en vue d'un réglage de largeur de fente autonome de sorte que des implants avec des épaisseurs de matériau différentes peuvent être introduits dans la fente de découpe (4) pour un court-circuit d'électrode.

7. Instrument chirurgical d'implant selon l'une quelconque des revendications 1 - 5, **caractérisé en ce que** les deux branches d'instrument (2) peuvent être déplacées ou réglées par l'intermédiaire d'un mécanisme d'actionnement afin de modifier de manière définie la largeur de fente de découpe.

8. Instrument chirurgical d'implant selon l'une quelconque des revendications précédentes, **caractérisé en ce que** les deux électrodes (10) et/ou les deux branches d'instrument (2) sont orientées l'une par rapport à l'autre sensiblement de manière à présenter une forme de V de sorte que la fente de découpe (4) réalisée de manière intercalée se rétrécit en continu dans le sens proximal ou en une forme courbe convexe.

9. Instrument chirurgical d'implant selon l'une quelconque des revendications précédentes, **caractérisé en ce qu'**un dispositif de précontrainte est prévu au niveau de la tête d'instrument (1), qui précontraint les unes contre les autres les électrodes (10) et/ou les branches d'instrument (2) avec une forme de précontrainte définie de sorte qu'un matériau d'implant introduit de manière intercalée est compressé ou serré de manière automatique par l'opération d'introduction elle-même.

10. Instrument chirurgical d'implant selon l'une quelconque des revendications précédentes, **caractérisé en ce qu'**un système d'identification de qualité de contact électrique et/ou électronique est prévu, qui exécute, de préférence en présence d'un actionnement de découpe de l'instrument et de manière chronologique avant l'exposition en résultant des électrodes à l'action d'un courant continu de puissance ou séparément et indépendamment d'un actionnement de découpe, une séquence test de la qualité du contact.

11. Instrument chirurgical d'implant selon la revendication 10, **caractérisé en ce que** la séquence test prévoit de soumettre les électrodes (10) à l'action d'un courant de test inférieur au courant continu de puissance ou de soumettre les électrodes (10) au courant continu de puissance toutefois avec des impulsions de test présentant une durée inférieure à la durée des impulsions de découpe afin de déterminer respectivement la résistance de contact entre les électrodes (10) respectives et l'implant/le tronçon d'implant de préférence selon le principe de mesure à quatre conducteurs.

12. Instrument chirurgical d'implant selon la revendication 11, **caractérisé en ce que** le système d'identification de qualité de contact électrique/électronique émet, dans le cas d'une valeur inférieure à une valeur limite, un signal d'avertissement et/ou bloque l'exposition qui suit au courant continu de puissance, et/ou **en ce que** le système d'identification de qualité de contact électrique/électronique émet, en cas d'atteinte ou de dépassement de la valeur limite, un signal d'acceptation et/ou autorise l'exposition qui suit à un courant continu de puissance.

13. Instrument chirurgical d'implant selon l'une quelconque des revendications précédentes 11 et 12, **caractérisé en ce que** le système d'identification de qualité de contact électrique/électronique adapte l'intensité du courant continu de puissance amené pour l'opération de découpe d'implant selon le résultat de la détermination concernant la résistance de contact.

14. Instrument chirurgical d'implant selon l'une quelconque des revendications 10 - 13, **caractérisé en ce que** le système d'identification de qualité de contact électrique/électronique vérifie dans la séquence test si le courant continu de puissance requis pour faire fondre l'implant ou le tronçon d'implant provoque un réchauffement de l'instrument sous la capacité de charge thermique des conduits d'amenée et/ou des électrodes.

15. Instrument chirurgical d'implant selon l'une quelconque des revendications précédentes, qui fonctionne en courant continu, avec une tête d'instrument (1), au niveau de laquelle au moins deux branches longitudinales d'instrument (2) se faisant face sont disposées, lesquelles définissent entre elle une fente de découpe (4) s'étendant dans le sens longitudinal de l'instrument, laquelle est adaptée pour recevoir de manière intercalée un implant ou un tronçon d'implant (18) électroconducteur, **caractérisé en ce que** les branches d'instrument (2) ou les électrodes (10) sont fabriquées à partir d'un matériau résistant à la température, dont l'énergie de fusion spécifique ajustée à la résistance est supérieure à 5, 99 $10^{-15}$ J $m^{-4}$ $\Omega^{-1}$.

100 - 150 A
20 - 40 V
+ Kontakt Messfunktion

Fig. 1

EP 2 967 712 B1

**Variante 1**

**„rigide Elektroden"**

**Fig. 2**

## Variante 2

**„federelastische Elektroden"**

**Fig. 3a**

EP 2 967 712 B1

## Variante 2

**Fig. 3b**

EP 2 967 712 B1

Variante 3

Elektroden mit Gelenke

Fig. 4a

EP 2 967 712 B1

Variante 3

**Fig. 4b**

EP 2 967 712 B1

**Funktionsprinzip**

**„Kontaktschneiden"**

**Fig. 5**

Fig. 6

abgerundet/kugelförmig

6a

2e

6b

2e

2

18

2

scharfkantig/spitz

6a

2

2e

18

2e

2

6b

Leiter 1    Elektrode 1    Metall-stent    Elektrode 2    Leiter 2

Kontakt 1    Kontakt 2

$R_{L1}$    $R_{E1}$    $R_{K1}$    $R_M$    $R_{K2}$    $R_{E2}$    $R_{L2}$

**e**

[$10^{15}$ J m$^{-4}$ Ω$^{-1}$]

e (Kupfer) = 210    e (Kupfer) = 210

e (Stahl unleg.) = 39,0    e (Stahl unleg.) = 39,0

?    e (Nickeltitan) = 4,86    ?

**Strompfad**
(unskaliert)

Eigenschaften im Kontaktbereich sind stark
von der spezifischen Greifsituation abhängig

**Stromdichte**

Metallstent

Zuleitung und Elektrode    An der
Kontaktstelle    An der
Kontaktstelle    Zuleitung und Elektrode

**Strompfad**
(unskaliert)

Fig. 7

**Fig. 8**

Fig. 9

$I_{gross,}$ regelbar

$I_{klein,}$ konstant

amp

amp

amp

Fig. 10a

Fig. 10b

Fig. 10c

Fig. 10d

Fig. 10e

Volumenelement

Stromfluss

Fig. 11

Elektrode

Zu schneidendes
Metall

Elektrode

Fig. 12

$R_{L1}$  $R_{E1}$  $R_{K1}$  $R_M$  $R_{K2}$  $R_{E2}$  $R_{L2}$

Bereich geringer Stromdichte, Ohmscher Bereich (Strom fächert sich auf den gesamten Leitungsquerschnitt auf)

Bereich maximaler Stromdichte (Strom muss durch einen kleinen Kontaktpunkt) Übergangswiderstand zwischen zwei Materialien sowie Lichtbögen führen zu zusätzlichem thermischen Energieeintrag.

Bereich hoher Stromdichte, Ohmscher Bereich: Strom kann sich nur begrenzt auffächern, nimmt vorzugsweise die kürzeste Verbindung zwischen beiden Kontaktpunkten)

Elektrode

Zu schneidendes Metall

Elektrode

Fig. 12

EP 2 967 712 B1

Elektrode

Initial Erwärmung vor allem an den
Kontaktstellen (hoher Übergangswiderstand
sowie hohe Stromdichte)

Erhöhung des Ohmschen Widerstandes im
Materialvolumen durch erhöhte Temperatur

Verstärkungseffekt des Energieeintrages
durch Temperaturanstieg sowie
Volumendurchwärmung durch hohe
Stromdichte führt zur schnellen Erreichung
der Schmelztemperatur entlang des Strompfades

Zu schneidendes
Metall

Elektrode

**Fig. 13**

EP 2 967 712 B1

| Material | Spezifischer Widerstand in Om | Linearer Widerstand-Temperatur-koeffizient in 1/K | Schmelz-punkt | Spez. Wärme-kapazität in kJ kg$^{-1}$ K$^{-1}$ | Spez. Gewicht in kg m$^{-3}$ | Spez. Schmelz-energie in J mm$^{-3}$ (bei 38°C) | Adjustiert um Widerstands-faktor in 10$^{15}$ J m$^{-4}$ O$^{-1}$ |
|---|---|---|---|---|---|---|---|
| Silber | 1.59 10$^{-8}$ | 0.0038 | 961 | 0.235 | 10497 | 2.28 | 143 |
| Kupfer | 1.68 10$^{-8}$ | 0.0039 | 1064 | 0.382 | 8960 | 3.51 | 210 |
| Gold | 2.44 10$^{-8}$ | 0.0034 | 1084 | 0.130 | 19290 | 2.62 | 107 |
| Wolfram | 5.6 10$^{-8}$ | 0.0045 | 3422 | 0.134 | 19000 | 8.62 | 154 |
| Eisen | 1.0 10$^{-7}$ | 0.005 | 1536 | 0.452 | 7500 | 5.08 | 50.8 |
| Stahl unlegiert | 1.43 10$^{-7}$ | | 1493 | 0.490 | 7800 | 5.56 | 39.0 |
| Titan | 4.2 10$^{-7}$ | | 1668 | 0.522 | 4505 | 3.83 | 9.12 |
| Edelstahl | 6.9 10$^{-7}$ | 0.0009 | 1147 | 0.477 | 7800 | 4.13 | 5.99 |
| Nickel-Titan (Martensit) | 7.6 10$^{-7}$ | | 1310 | 0.45 | 6450 | 3.69 | 4.86 |
| Muskelgewebe | 2 | | | | | | |
| Trinkwasser | 20 bis 2000 | | | 4.182 | | | |
| Destilliertes Wasser | 180000 | | | | | | |

Fig. 14

**IN DER BESCHREIBUNG AUFGEFÜHRTE DOKUMENTE**

**In der Beschreibung aufgeführte Patentdokumente**

- WO 2008090003 A1
- DE 102007003838 A1
- US 3431384 A
- WO 2008040485 A2
- US 2012265196 A1
- US 2013030428 A1
- EP 2392282 A1
- DE 102007003838